# EUROPEAN PATENT SPECIFICATION

(11) **EP 4 299 043 B1**
(45) Date of publication and mention of the grant of the patent: **30.09.2026**
(21) Application number: 23181742.0
(22) Date of filing: 27.06.2023
(51) Int. Cl.: A61F 9/008

(54) **APPARATUS FOR OPHTHALMIC TREATMENT**
VORRICHTUNG ZUR OPHTHALMISCHEN BEHANDLUNG
APPAREIL DE TRAITEMENT OPHTALMIQUE

(30) Priority: 28.06.2022 US 202217809468
(43) Date of publication of application: 03.01.2024
(73) Proprietor: NORLASE ApS, 2750 Ballerup (DK)
(72) Inventor: GØTH, Bjarke, 4000 Roskilde (DK); SKOVGAARD, Peter M. W., 3460 Birkerød (DK); FAVA, Greg, Redwood City, 94062 (US)
(74) Representative: Guardian IP Consulting I/S

(56) References cited:
- WO-A1-2014/049132
- WO-A1-2014/174148
- US-A1- 2007 129 775
- US-A1- 2008 015 553
- US-A1- 2011 245 816
- US-A1- 2013 304 046
- US-A1- 2021 154 048

## Description

### Field of the invention

The present invention relates to an apparatus for ophthalmic treatment, in particular photothermal ophthalmic treatment by causing absorption of light by structures of a subject's eye, such as melanin or blood, in particular for photocoagulation or photo-thermal stimulation to treat retina and glaucoma disease. The present disclosure further relates to a method for providing a visible aiming target scheme to deliver a selected pattern to the retina of a subject.

### Background

Laser-based ophthalmic treatment, such as laser photocoagulation, is a well-established practice within ophthalmology for treating diseases such as glaucoma, diabetic retinopathy, wet age-related macular degeneration (AMD), and other retinal diseases.

In this context it is generally desirable to provide an apparatus for ophthalmic treatment that is easy to use for the operator. It is further generally desirable to provide an apparatus for ophthalmic treatment that can be manufactured in a cost-efficient manner. It is further generally desirable to provide a compact and versatile solution. It is further generally desirable to reduce the complexity such an apparatus. It is further generally desirable to provide an apparatus for ophthalmic treatment that is durable.

Laser coagulation requires the exposure of the retina to visible laser light and relies on the selective absorption in melanin and hemoglobin, typically at a wavelength range of 514-577 nanometers (nm), to achieve vessel coagulation. Laser photocoagulators focus on this range of wavelengths and aim to provide small spot sizes, typically 50 to 500 µm, adjustable power, e.g. in the range 0-2.0 W, and long depth of focus to increase treatment precision and quality with less heat, to minimize effects to surrounding tissues. In the course of treatments, physicians will regularly set and update parameters dictating the laser energy to be delivered. These parameters can include power, pulse duration, and laser spot size, among other examples.

Commonly, slit lamps are used for delivering the laser energy to the patient's eye. In these systems, the patients typically sit up in an examination chair, rest their chin on a chin rest, and place their forehead against a forehead band, both of which keep the patient's head in place during the procedure.

Another common device is a Laser Indirect Ophthalmoscope (LIO), which is a head mounted device, worn by the doctor to deliver laser energy into a patient's eye. During procedures using the LIO, the doctor moves the laser console for generating the laser energy, which is positioned on a cart or table, to be in the proximity of the patient who is usually in a supine position. The doctor then walks around the patient to deliver the laser energy to the desired portions of the retina.

Photothermal ophthalmic therapy typically involves directing a treatment beam, in particular a treatment laser beam, onto a location on a structure in the subject's eye, in particular the retina. Photothermal ophthalmic treatment is typically performed point-by-point (single spot delivery), where each individual dose of radiation is directed and delivered to a selected target position on the structure to be treated. The treatment beam may be individually directed to the respective selected treatment positions by a physician. To this end, an aiming laser beam at a visible wavelength different from the treatment wavelength, is typically used in conjunction with, in particular prior to, a treatment laser beam. The aiming laser beam, sometimes also referred to as a pilot beam, indicates (targets) the treatment location on the retina or other structure, which is subsequently to be treated by the treatment laser beam. Each dose at each treatment location is individually initiated by the physician, e.g. by activating a foot switch.

Point-by-point (single spot) treatment tends to be a lengthy procedure, in particular when a large number of treatment locations are needed. This increases treatment costs, may cause physician fatigue as well as discomfort for the patient being treated. Ultimately, this may cause a prematurely terminated treatment, in which case the patient is left under-treated or needs to return at a later date to finish treatment.

Several approaches have been suggested in an attempt to reduce treatment times. One such approach involves multi-spot treatment (pattern Laser treatment) where the aiming beam is controlled to indicate multiple treatment locations and where the physician can activate a sequence of doses, which are then automatically directed to multiple treatment locations.

To this end, various approaches have been suggested for indicating multiple treatment locations by the aiming beam.

US 7766903 suggests a system where a visible alignment pattern having at least two separated spots is projected onto the retina. By triggering a laser subsystem, doses of laser energy are automatically provided to at least two and up to 56 treatment locations coincident with the alignment spots. To this end, a scanner is used to sequentially move an alignment beam from spot to spot on the retina and to move a treatment laser beam from location to location on the retina. However, during the alignment procedure, the sequential display of multiple separated spots may result in visible flicker and it may increase the time required for displaying the alignment pattern, in particular when many treatment locations are to be treated. In addition, by illuminating every treatment location, a large amount of aiming light must be projected to the retina. As adjacent aiming spots scatter light at the target tissue, the individual spots become less prominent and large patterns are less distinct. This may negatively affect the physician's view of the structure to be treated.

US 2021/0154048 suggests projecting one or more lines which form a stationary pattern that overlies all the spots of the spot pattern to be treated. While this approach mitigates some of the disadvantages of sequentially illuminating separated spots, this prior art method involves illuminating a relatively large area, which may negatively affect the physician's view of the structure to be treated. Moreover, the method still requires directing the aiming beam along a long path that intersects all treatment locations to be treated. The long length of such path may result in flickering of the aiming pattern as well as a lot of aiming light that may be blinding or uncomfortable for the physician or the patient.

WO 2014/174148 suggests an outline mode, wherein the aiming beam draws a frame around the area where the treatment pulses are to be delivered. While this allows the physician to have a clear view of the retina inside the frame at larger spot spacing, it is less desirable at smaller spot spacing because the lines are so close together within the frame that it is difficult to determine the area to be treated because of light scattering at the target tissue. It also increases the requirements on the optical system to direct light to/from a larger field of view and it still negatively affects the physician's view of the immediate surroundings of the structure to be treated. In addition, it would still be desirable to reduce the amount of aiming light projected to the retina, so as to reduce the risk that the aiming light negatively affects the physician's view of the structure to be treated.

US 2008/0015553 A system and method for delivering therapeutic laser energy onto selected treatment locations of the retina following a predetermined spatial distribution pattern using one single laser beam. A beam steering mechanism and control system delivers the laser energy sequentially to treatment locations forming a pre-selected treatment layout pattern. According to this prior art method, an aiming beam pattern is positioned by an operator allowing him to clearly identify a retinal area where the laser energy will be delivered to treatment locations.

### Summary

In view of the above it remains desirable to provide an apparatus for ophthalmic treatment that mitigates one or more of the disadvantages of prior art systems or that at least can serve as an alternative.

According to a first aspect, disclosed herein is an apparatus for photothermal ophthalmic treatment comprising:
a) a treatment light source for producing a treatment beam,
b) a scanning module configured for delivering the treatment beam onto separate treatment locations within a treatment target area on a structure, in particular the retina, of a subject's eye,
c) an aiming light source for producing an aiming beam,

wherein the scanning module is configured for delivering the aiming beam to create a visible outline pattern on the structure of the subject's eye, the visible outline pattern being indicative of a periphery of said treatment target area. The apparatus is characterized in that the visible outline pattern includes one or more pattern elements, each pattern element perceivable as moving along the periphery,
that the scanning module is configured to cause an aiming beam path of the aiming beam to repeatedly scan along a scan path extending along the periphery, and
that the apparatus is configured to selectively activate and deactivate the aiming beam so as to only illuminate respective portions of the scan path during each scan along the scan path, so as to create a perceivable beta movement of the visible outline pattern along the periphery.

In some embodiments, each pattern element is a line having two endpoints, in particular a line that is shorter than the periphery. In particular, the periphery may be represented by a closed perimeter line surrounding all treatment target locations within a target area, such that all target treatment locations of said target area are located inside or, optionally, on the perimeter line. When a pattern element of the visible outline pattern is a line, the line may correspond to a segment of the perimeter line. The line may correspond to, in particular extend along, or even coincide with, a portion of the periphery, in particular a portion of the perimeter line.

In some embodiments, the line of the visible outline pattern is perceivable as moving along the periphery in a direction between a trailing end and a leading end of the line. The line may be a straight line or a curved line; it may change shape during the course of the perceived movement along the periphery, in particular in accordance to a local shape of the periphery along a movement path of the line of the visible outline pattern.

The visible outline pattern may include two, three, four or even more separate pattern elements which are spaced apart from each other, i.e. such that the pattern elements do not overlap each other. The separate pattern elements forming the visual outline pattern may together only cover a portion of the periphery. All pattern elements may be perceivable as moving along the periphery in a common direction at a common perceived speed, or their perceived movement may be in different directions and/or at different speeds.

In some embodiments, the apparatus is configured to select the number of pattern elements of the visible outline pattern responsive to a treatment parameter indicative of a property of the delivery of the treatment beam, in particular responsive to a user-selected treatment parameter. For example, when the apparatus is configured to deliver the treatment beam to treatment locations defining a user-selected treatment pattern, the apparatus may be configured to select the number of pattern elements of the visible outline pattern responsive to the user-selected treatment pattern or a property thereof, such as the shape or size of the treatment pattern, e.g. the number of treatment locations in the treatment pattern, etc. Other examples of properties of the delivery of the treatment beam may include a pulse duration or another property of the treatment beam itself, e.g. a beam intensity.

The scanning module is configured to cause an aiming beam path of the aiming beam to repeatedly scan along a scan path that extends along the periphery. The apparatus is configured to selectively activate and deactivate the aiming beam so as to only illuminate respective portions of the scan path during each scan along the scan path, so as to create a perceivable beta movement of one or more pattern elements of the visible outline pattern along the periphery. Activation and deactivation of the aiming beam may be implemented by selective shuttering the aiming beam, i.e. by using a shutter, or by selectively switching the aiming light source on and off. Activation and deactivation by selectively turning the aiming light source on and off reduces the complexity of the apparatus.

In some embodiments, the apparatus is configured:
- during a first scan of the aiming beam path along a scan path extending along the periphery, to selectively activate and deactivate the aiming beam so as to only illuminate a first portion of scan path,
- during a second scan of the aiming beam path along said scan path, subsequent to the first scan, to selectively activate and deactivate the aiming beam so as to only illuminate a second portion of the scan path, the second portion being displaced relative to the first portion along the direction of the scan path.

The scan path may coincide with the periphery or it may extend adjacent to the periphery, e.g. immediately adjacent or parallel with, and displaced from the periphery.

The periphery may surround, in particular completely surround the treatment target area. In some embodiments, the visible outline pattern may intersect one or more of the treatment locations, in particular treatment locations lying close to or even on the periphery of the target area. In other embodiments, the visual outline pattern, is spaced apart from all treatment locations. For example, the periphery may be a closed perimeter line defining the target area as the area enclosed by the closed perimeter line. The closed perimeter line may be a part of the target area. Accordingly, the closed perimeter line may extend between two or more outermost treatment locations of the treatment area. The visual outline pattern may extend along the periphery, e.g. coinciding with or extending adjacent, such as immediately adjacent, e.g. immediately outside or immediately inside, the periphery.

The treatment and aiming light sources may be separate light sources or a single light source operable to selectively produce a treatment beam and an aiming beam of a different visible wavelength. The treatment light source may be a treatment laser source and the treatment beam a treatment laser beam. Similarly, the aiming light source may be an aiming laser source and the aiming beam an aiming laser beam. In some embodiments, the treatment laser source may be a treatment laser diode. Similarly, the aiming laser source may be an aiming laser diode. The treatment light source is generally configured to output light at a wavelength and output power that is therapeutically efficient. The aiming light source is generally configured to output light at a wavelength and output power that is suitable for good visibility and that preferably has not therapeutic or adverse effect on the structure being illuminated by the aiming light. Accordingly, the aiming beam has generally a different wavelength than the treatment beam. The aiming beam has typically a lower power than the treatment beam.

The scanning module may include separate components for delivery the aiming and treatment beams, respectively. Alternatively, the scanning module may include one or more components configured for delivering the treatment and the aiming beam. The scanning module may include one or more scanning mirror, e.g. 2D scanning mirror or two 1D scanning mirrors.

Each treatment target location may have the form of a spot having a predetermined and/or user-selectable spot size. During operation, responsive to activation of an activation unit, such as a foot switch, by the physician, the apparatus may control the treatment light source and the scanning module so as to automatically direct the treatment beam to some or all of the treatment target locations in the treatment target area so as to deliver respective doses of light energy, in particular laser energy, to said some or all of the treatment target locations. To this end, the scanning module may sequentially direct the treatment beam to respective ones of the treatment locations within the target treatment area and the apparatus may selectively activate and deactivate the treatment beam so as to deliver doses of light energy to the respective treatment locations.

The apparatus may comprise a control module. The control module may be configured for controlling operation of the treatment light source, the aiming light source and the scanning module. The control module may be accommodated in the same housing as one or all of the treatment light source, the aiming light source and the scanning module.

The apparatus may comprise a user-interface configured to provide functionality for user-selection of a treatment pattern. The treatment pattern defines relative locations of the plurality of separate treatment locations relative to each other and/or relative to a common reference coordinate system. In particular, such user-interface may be configured such that the user can select and adjust the number of spots, the pattern (e.g. squares, circles or arcs), the distance between spots, the power of the treatment laser delivered to each spot, the pulse length in each treatment spot etc.

Accordingly, the apparatus may comprise a user device, such as a tablet computer, a personal computer, or another type of handheld, portable, wearable or otherwise mobile computing device, etc. The user device may be configured to provide the user-interface and it may be communicatively coupled, e.g. via a wire or wireless connection, with the control module. The apparatus may further comprise a user activation device, such as a foot switch or the like.

Preferably, the visual outline pattern covers between 20% and 90%, such as between 30% and 80% of the periphery. Preferably, the one or more pattern elements of the visible outline pattern move along the entire periphery, in particular such that each part of the periphery is traversed by the perceived movement of at least one of the one or more pattern elements of the visible outline pattern. Preferably, each part of the periphery is traversed repeatedly by the perceived movement of at least one of the one or more pattern elements of the visible outline pattern.

In some embodiments, the visual outline pattern comprises or even consists of N distinct line elements. The line elements have respective lengths *lᵢ, i* = 1, ... , *N*, which are preferably selected such that $0.2 \leq \frac{{\sum }_{i = 1}^{N} {l}_{i}}{L} \leq 0.8$, where L denotes the length of the periphery. In some embodiments, the line elements of the visual outline pattern have all the same lengths. The line elements are preferably separate and spaced apart line elements that are not extensions of each other or otherwise overlap with each other.

In some embodiments, the apparatus comprises a diagnostic instrument - e.g. a slit-lamp or operating microscope apparatus - and an adapter unit, which may be attached to or be an integral part of said diagnostic instrument. The diagnostic instrument is configured to emit illumination light from an illumination output along a free-air illumination output path towards a target area for ophthalmic treatment. The diagnostic instrument is further configured to receive light from the target area along a free-air viewing path and to provide a magnified view of the target area. The adapter unit comprises the scanning module and, preferably, one or both of the treatment light source and the aiming light source.

In some embodiments, the apparatus comprises a diagnostic instrument - e.g. a slit-lamp or operating microscope apparatus - having a housing where the treatment light source, the scanning module and the aiming light source are partly or completely integrated into the housing. For example, the treatment light source, the scanning module and the aiming light source may be completely integrated into the slit-lamp illumination housing or slit-lamp viewing scheme. Similarly, the treatment light source, the scanning module and the aiming light source may be embodied as an adapter for, as an integrated part of, a surgical microscope for use in an operating room or other surgical environment. Using an apparatus as described herein in the operating room may improve the amount of time to complete the laser procedure and eliminate the need for a disposable endoprobe to complete the laser photocoagulation.

In some embodiments, the apparatus may comprise a body worn, e.g. head-mounted housing. In particular, the apparatus may be a Laser Indirect Ophthalmoscope (LIO).

The present disclosure relates to different aspects including the apparatus for ophthalmic treatment described above and in the following, corresponding apparatus, systems, methods, and/or products, each yielding one or more of the benefits and advantages described in connection with the first mentioned aspect, and each having one or more embodiments corresponding to the embodiments described in connection with the first mentioned aspect and/or disclosed in the appended claims.

In particular, according to one aspect, the present disclosure relates to a method for providing a visible aiming pattern on a structure, in particular the retina, of a subject's eye, the method comprising delivering an aiming beam to create a visible aiming pattern on the structure of the subject's eye, the aiming pattern being a visible outline pattern indicative of a periphery of a treatment target area. The method is characterized in that the visible outline pattern includes one or more pattern elements, each pattern element perceivable as moving along the periphery, and that the method comprises causing an aiming beam path of the aiming beam to repeatedly scan along a scan path extending along the periphery, and selectively activating and deactivating the aiming beam so as to only illuminate respective portions of the scan path during each scan along the scan path, so as to create a perceivable beta movement of the visible outline pattern along the periphery. The scope of the invention is defined by the following claims.

### Brief description of the drawings

Fig. 1 is a schematic diagram of an apparatus for photothermal ophthalmic treatment.
Fig. 2 is a schematic side view of an apparatus for photothermal ophthalmic treatment the laser treatment system according to an embodiment in which a pattern-scanning laser device of the apparatus is attached to a slit lamp;
Fig. 3 is a schematic front view of an apparatus for photothermal ophthalmic treatment according to an embodiment in which the pattern-scanning laser device is attached to a body-mounted laser indirect ophthalmoscope;
Fig. 4A is a schematic diagram of an embodiment of the pattern-scanning laser device;
Fig. 4B is a perspective view of the pattern-scanning laser device with a portion of a housing of the device removed to reveal internal components;
Fig. 5 is a block diagram of an embodiment of a laser module of the pattern-scanning laser device;
Fig. 6 is a schematic diagram of the laser module showing components in more detail;
Fig. 7 is a perspective schematic view of an exemplary scanning module of the pattern-scanning laser device comprising a single two-dimensional scanning mirror;
Fig. 8 is a perspective view of an exemplary scanning module comprising two one-dimensional scanning mirrors;
Fig. 9 is a side view showing an exemplary F-theta lens of delivery optics for the pattern-scanning laser device;
Fig. 10 is a side view of the delivery optics showing the F-theta lens, a spot-size selector module and a focusing lens;
Fig. 11 is a schematic diagram of an embodiment of an apparatus for photothermal ophthalmic treatment showing components of the pattern-scanning laser device, an activation unit and a mobile computing device;
Fig. 12 is a sequence diagram illustrating a process by which an embodiment of an apparatus for photothermal ophthalmic treatment delivers provides a visible outline pattern and, subsequently, patterned treatment laser energy to an eye of a patient.
FIG. 13 shows an example of a graphical user interface;
FIGs. 14A-D illustrate examples of treatment patterns and associated visible outline patterns;
FIGs. 15A and 15B show examples of treatment patterns in the form of one or more circular arcs;
FIGs. 16A-F show examples of visible outline patterns that outline the periphery of a treatment target area which includes a treatment pattern in the form of treatment locations arranged on a grid; and
FIGs. 17A-B illustrate an example of how a visible outline pattern of pattern elements that are perceived to move along the periphery of a treatment target area is generated by the scanning module.
FIGs. 18A-D show further examples of visible outline patterns that outline the periphery of a treatment target area.

### DETAILED DESCRIPTION

Various aspects disclosed herein will be described more fully hereinafter with reference to the accompanying drawings, in which illustrative embodiments are shown. This invention may, however, be embodied in many different forms and should not be construed as limited to the embodiments set forth herein; rather, these embodiments are provided so that this disclosure will be thorough and complete, and will fully convey the scope of the invention to those skilled in the art.

As used herein, the term "and/or" includes any and all combinations of one or more of the associated listed items. Further, the singular forms and the articles "a", "an" and "the" are intended to include the plural forms as well, unless expressly stated otherwise. It will be further understood that the terms: includes, comprises, including and/or comprising, when used in this specification, specify the presence of stated features, integers, steps, operations, elements, and/or components, but do not preclude the presence or addition of one or more other features, integers, steps, operations, elements, components, and/or groups thereof. Further, it will be understood that when an element, including a component or a subsystem, is referred to and/or shown as being connected or coupled to another element, it can be directly connected or coupled to the other element or intervening elements may be present.

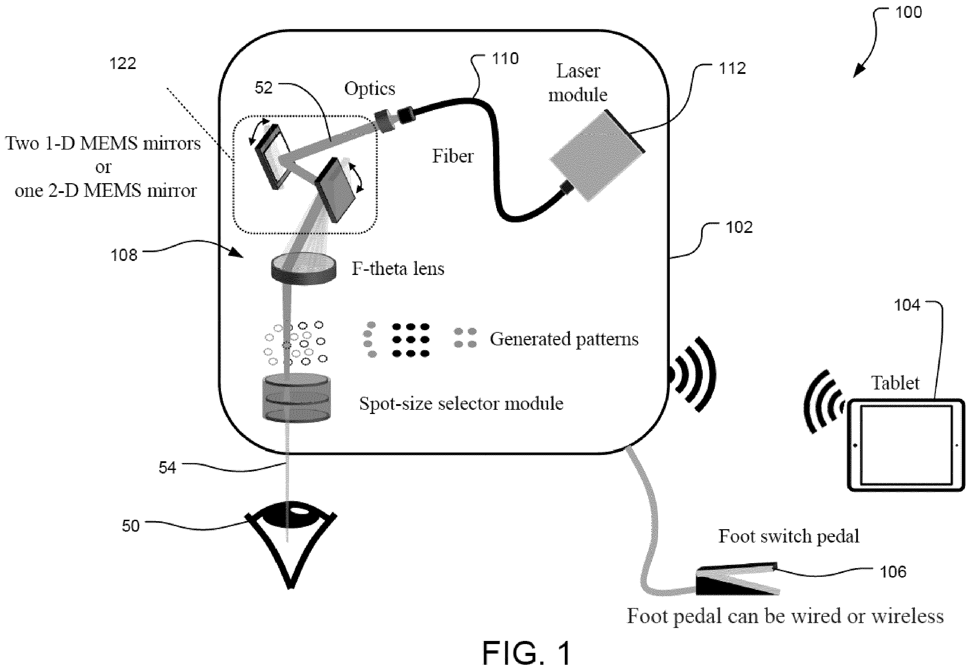

Fig. 1 is a schematic diagram of apparatus for photothermal ophthalmic treatment, in particular a multi-spot laser treatment system 100. In general, the multi-spot laser treatment system 100 delivers patterned laser energy 54 to the retina of an eye 50 of a patient. In particular, the multi-spot laser treatment system 100 is configured to selectively deliver an aiming laser beam and a treatment laser beam. Each of the aiming laser beam and the treatment laser beam are scanned across a part of the retina so as to deliver respective scanned aiming and treatment patterns, respectively.

The multi-spot laser treatment system 100 delivers the treatment laser energy in a predetermined two-dimensional pattern of treatment locations in the form of spots. The pattern of spots is based on the pathology or treatment for the patient. The presently disclosed system can deliver treatment laser energy in a pulse train such as discrete pulses known as micropulses, each pulse having a duration ranging from 10-1000 microseconds (µs). In addition to delivering the treatment laser energy, the multi-spot laser treatment system 100 delivers an aiming laser beam so as to form a visible aiming pattern on the retina, in particular a visible outline pattern that indicates the outline of a treatment target area on the retina as described herein.

The multi-spot laser treatment system 100 includes a pattern-scanning laser device 102, an activation unit 106 and a mobile computing device 104.

The pattern-scanning laser device 102 generates the patterned laser energy 54, in particular the treatment pattern and the visible outline pattern, and it includes a laser module 112, a fiber optic cable or freespace optical connection 110, a scanning module 122, and delivery optics 108.

The laser module 112 produces the aiming laser beam and the treatment laser beam. The laser module 112 emits treatment laser energy according to certain user-provided parameters such as desired power, overall duration, and parameters related to pulsed laser energy including pulse envelope duration, peak power, and micropulse duration and interval, among other examples. The treatment laser beam and the aiming laser beam produced by the laser module 112 are transmitted through the fiber optic cable 110 to the scanning module 122.

In the preferred embodiment, the laser module 112 produces treatment laser energy suitable for ophthalmic treatments, in particular treatment laser energy in a wavelength in the visible green wavelength range (e.g. 520 nm) with adjustable power output up to 2 Watts (W), with treatments typically being in the range of .05-1 W. A treatment direct diode of the laser module produces a collimated treatment beam, which is combined with a lower-power aiming beam (e.g. 1-5 mW) via a polarization combiner and directed through optics for beam manipulation and shaping and into a 50 µm core fiber 110 to be delivered to the scanning module 122. The laser module further produces an aiming beam, preferably at a lower power and a wavelength visible to the human eye, but such that it does not adversely affect the retina. In a preferred embodiments the aiming laser diode outputs visible red light.

The scanning module 122 produces the patterned laser energy 54 by directing the laser energy 52 produced by the laser module into the delivery optics 108 at different angles according to pre-determined patterns. In particular, the scanning module 122 produces patterned treatment laser energy by directing the treatment laser energy produced by the laser module into the delivery optics 108 at different angles according to pre-determined patterns of spots (e.g. each spot being associated with each reflected laser beam). Similarly, the scanning module 122 produces a visible outline pattern by directing the aiming laser beam produced by the laser module into the delivery optics 108 at different angles according to pre-determined pattern elements of a visible outline pattern as described herein, in the form of lines or other pattern elements that are perceivable by the physician observing the retina as moving along the periphery of the treatment target area.

Generally, in some embodiments, the scanning module and delivery optics are configured to deliver laser energy onto the retina as a spot. In order to deliver the treatment laser energy in the form of a treatment pattern including individual, spaced apart spots, the scanning module is preferably configured such that it is able to move the treatment laser beam very fast across the structure to be treated, and the laser module is preferably configured to be able to turn the treatment laser beam on and off very fast. Typically, a scanning module allows movement of the treatment laser beam from one spot location to another within less than 5 msec. e.g. less than 3 msec. such as within 2 msec. or less. Once the scanning module is resting in position, the treatment laser can be turned on for a short time to perform laser treatment at the current spot location, before the scanning module moves the treatment laser beam to the next treatment location of the treatment pattern.

Prior to delivering the treatment laser energy, the apparatus is configured to project a visible aiming pattern onto the retina. To this end, the scanning module and delivery optics are configured to deliver an aiming laser beam onto the retina as a spot. In order to deliver the aiming laser energy in the form of a visible outline pattern, the scanning module is preferably configured such that it is able to move the aiming laser beam very fast across the structure to be treated, and the laser module is preferably configured to be able to turn the aiming laser beam on and off very fast. As mentioned above, the scanning module may allow movement of the aiming laser beam from one spot location to another within less than 5 msec. e.g. less than 3 msec. such as within 2 msec. or less.

In some prior art systems, an aiming pattern is delivered as separate, spaced apart spots coinciding with the treatment locations of the treatment pattern. Accordingly, in order to deliver such a prior art alignment pattern, once the scanning module is resting in position, the aiming laser beam can be turned on for a short time, e.g. for about 2 msec. to illuminate a designated treatment spot at the current spot location, before the scanning module moves the aiming laser beam to the next target treatment location of the treatment pattern. Generally, the aiming laser beam is typically a low power laser beam, with optical powers of around 2 mW or less. This means that at any given spot location, the laser should be turned on long enough to make the spot visible for the physician. In case of a treatment pattern in the form of a 5 x 5 spot pattern, assuming a scanning time between spots of 2 msec and an illumination time of 2 msec. at each spot, the total time required to scan across the entire pattern and illuminate all 25 spots of the treatment pattern with the aiming laser beam is about 2 x 2 x 25 = 100 msec. This means that the spot pattern will be displayed with a repetition rate of only 10 Hz, only 10 times per second. However, for the human eye to see a stationary pattern, the frequency should preferably be above 50 Hz. The lower the frequency, the more the pattern will appear as flickering.

In embodiments of the apparatus disclosed herein, the aiming pattern is a visible outline pattern representing the periphery of the treatment area at which the treatment pattern is to be located. To this end, the scanning module may repeatedly scan the aiming beam along a scan path that extends along the periphery. Accordingly, the scan path may be a closed path, e.g. a square, rectangle, circle, etc, or it may be a line that is repeatedly scanned, e.g. in a reciprocating movement. In order to project a visible outline pattern, the aiming laser beam is activated / turned on while the scanning module is moving, i.e. while the illuminated spot delivered by the aiming laser beam is moving across the retina. Accordingly, there is no need to turn off the laser, wait until the scanning module is in position and then turn on the laser beam. The movement of the scanning module may have to decelerate and accelerate as the beam travels around corners, which will slow down the repetition rate. But even with this, the much simpler pattern of a circle, rectangle, square or the like will result in a much higher repetition rate, the projected outline will be perceived by human vision as an illuminated line. For example, when the scanning module is configured to move the aiming laser beam from one corner of a square contour to the other in 5 msec, the entire square contour can be outlined in 4 x 5 = 20 msec, thus allowing a repetition rate of 50 Hz, which will be perceived by a human eye as being a substantially constant illumination without flickering.

Further, repeatedly turning on and off the aiming laser beam while the scanning module scans the aiming laser beam along the scan path defining the visible outline pattern results in cutting the illuminated contour into individual spaced apart lines as described herein. By carefully controlling the timing (speed) of this modulation, the lines can be made to appear as moving along the scan path, as will be explained in more detail below. By illuminating only a portion of the outline, the total amount of aiming light can be reduced at a similar rate. This can lead to better visualisation by the physician and less visual discomfort for the patient.

With the outline pattern being different from the treatment pattern, the risk is reduced that an operator might confuse the aiming and treatment patterns with each other. In particular, a stationary aiming pattern, in particular when in the form of individual dots, may be confused with the actual treatment pattern of an ongoing treatment. Accordingly, the operator may be unsure if he/she has already performed (or is currently performing) the treatment when looking through the slitlamp.

Generally, the scanning module is preferably configured to repeatedly scan the aiming beam along the periphery of the treatment area at a scanning rate of 40 Hz or more, such as 50 Hz or more.

The scanning module includes one or more scanning mirrors, preferably MEMS scanning mirrors, each comprising a reflective surface and associated control circuitry such as actuators, which cause the reflective surfaces of the mirrors to reflect incoming laser energy at different angles, for example, by rotating the mirrors about an axis. The reflective surface includes a reflective coating (e.g. highly reflection (HR) Bragg coatings for 520 nm wavelengths or external optical HR coatings).

In the illustrated embodiment, the scanning module 102 includes two opposed one-dimensional scanning mirrors, the reflective surfaces of which each respectively rotate on a different axis. However, in other embodiments, the scanning module includes one two-dimensional scanning mirror, the reflective surface of which rotates on two different (e.g. mutually perpendicular) axes.

The delivery optics 108 output the patterned laser energy of the treatment and aiming laser beams to the patient's retina 50 (e.g. via a slit-lamp or LIO optics). In general, the delivery optics 108 are designed and configured to ensure beam quality and provide for adjusting the beam diameter via a motorized and wirelessly-controlled adjustment mechanism. In one example, the delivery optics 108 are designed to be capable of producing only specific laser beam spot sizes suitable for multi-spot ophthalmic laser treatments, such as spot sizes of 100 µm and 200 µm.

In some embodiments, the laser system may include separate laser modules for producing the treatment and aiming laser beams, respectively. Alternatively or additionally, the treatment and aiming laser beams may partly or completely be transmitted along different paths, e.g. involving different optical elements.

The activation device 106, which is part of the user interface of the multi-spot laser treatment system 100, is a device that receives user input via an activation mechanism (e.g. a switch or button) and in response sends activation signals to the scanning laser device 102 indicating that the laser energy should be emitted. The activation device 106 is typically a footswitch, and engagement with the activation mechanism includes compression of the footswitch by the user's foot, for example.

Preferably, the mobile computing device 104 is a medical-grade tablet computer. Alternatively, the mobile computing device 104 could be a smartphone device, laptop computer, or phablet computer (i.e., a mobile device that is typically larger than a smart phone, but smaller than a tablet), to list a few examples. In general, the mobile computing device 104 provides additional components of the user interface and generates parameter information indicating the user-provided parameters based on input received via the user interface and wirelessly (e.g. via Bluetooth or Bluetooth Low Energy connectivity) sends the parameter information to the pattern-scanning laser device 102. The mobile computing device 104 includes a user interface including a graphical user interface and/or voice control capability. In examples, the mobile computing device 104 allows doctors to adjust power level, pulse duration, period, pre-set treatment programs, via touch or voice, and receive audible feedback indicating selected parameters.

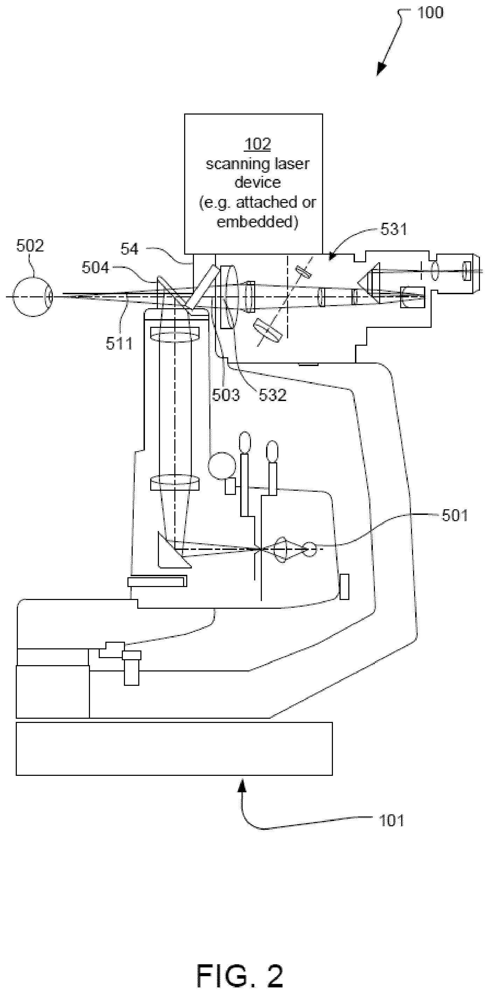

Fig. 2 schematically shows a slit lamp device 101 to which the present invention is applicable. The figure schematically shows the previously described pattern-scanning laser device 102 attached to or incorporated into the slit lamp 101. The slit lamp 101 includes a magnifying optical device 531, such as a microscope or zoom telescope, configured to receive light at a viewing input 532 along a viewing path 503 from a target area. The central part of the slit lamp 101 includes a white light source 501 that is used to illuminate a target area in the eye 502 of the patient. This white light is directed, by means of a mirror 504, onto an illumination output path 511 that coincides with the optical viewing path 503 of the operator at the designed focal point of the diagnostic instrument at the target area. In the same fashion, the light 54 from the laser is directed towards the target area along a treatment/aiming beam path such that it coincides with the viewing path, at least at the target area.

In one embodiment, the pattern-scanning laser device 102 is attached to an existing (e.g. legacy) slit lamp device 101 via an attachment mechanism.

In another embodiment, the pattern-scanning laser device 102 is fully integrated into the slit lamp device 101.

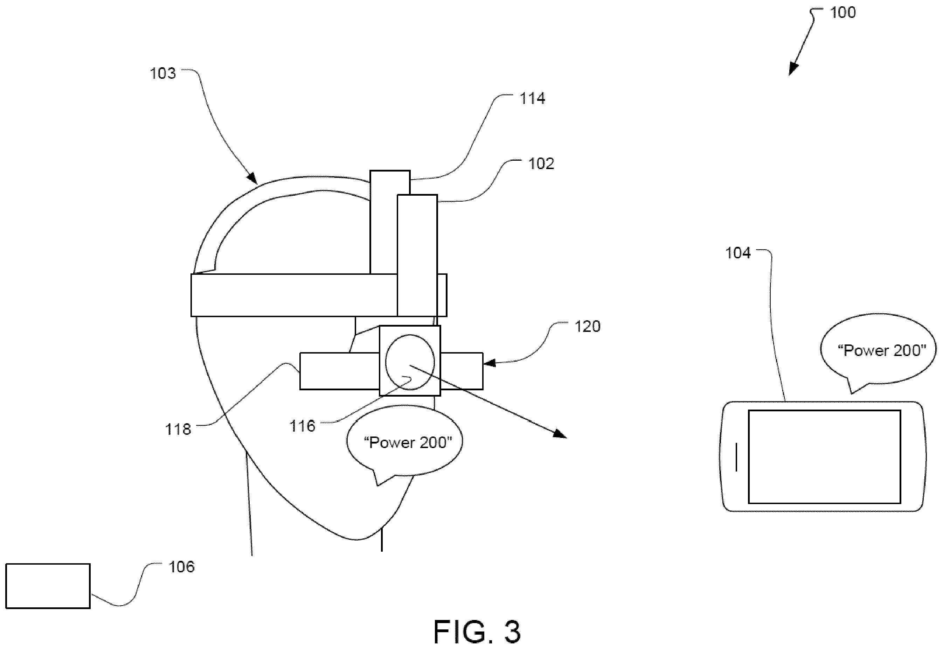

Fig. 3 is an illustration of a multi-spot laser treatment system 100 according to an embodiment in which the scanning laser device 102 is attached to a body-mounted LIO system. In general, the body-mounted LIO system delivers the patterned laser energy 54 to an eye 50 of a patient. A user of the LIO system is typically a physician such as an ophthalmologist.

The body-mounted LIO system includes a binocular indirect ophthalmoscope 120, which is an optical device for examining the inside of the eye 50 of the patient. The binocular indirect ophthalmoscope 120 includes an illumination unit 114 for providing white light and an optical system including a viewing aperture 118 and an exit aperture 116 from which the laser energy is emitted (which is also an entrance aperture for image information e.g. for viewing the patient's eye).

The body-mounted LIO system includes a wearable assembly 103, which secures the body-mounted LIO system, including the pattern-scanning laser device 102, to the user's body via one or more wearable objects such as a headset, a utility belt, or a backpack, among other examples. In the illustrated example, the wearable assembly 103 comprises only a headset, which is worn on the user's head.

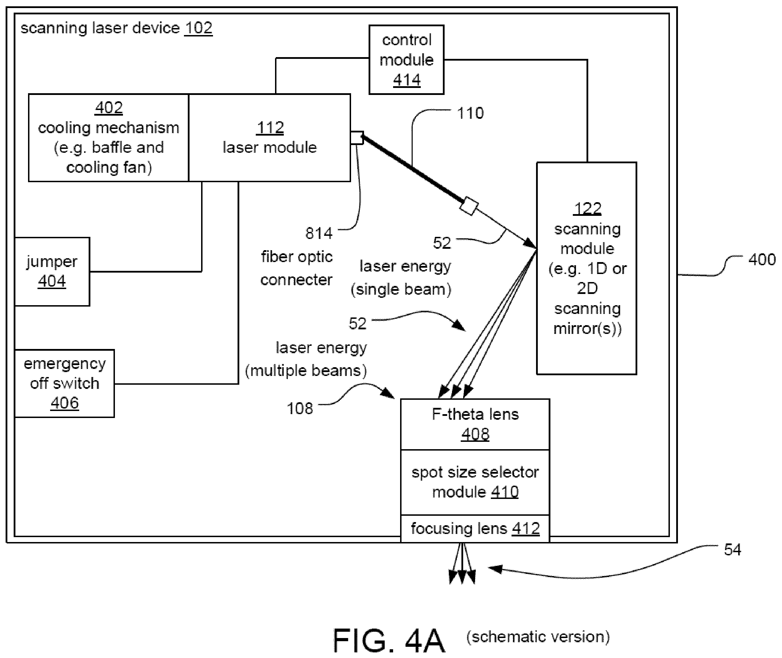

Fig. 4A is a schematic diagram of the pattern-scanning laser device 102.

The pattern-scanning laser device 102 includes the laser module 112, fiber optic cable 110, scanning module 122, and delivery optics as previously described.

Now, however, additional components are shown, including a cooling mechanism 402, a jumper 404, an emergency off switch 406, a control module 414, an F-theta lens 408, spot size selector module 410, and focusing lens 412 of the delivery optics 108, and a compact housing 400.

The compact housing 400 provides an enclosure for all of the components of the scanning laser device 102, which is designed to have an optimal size, shape and weight to be securely mounted to any existing slit-lamp or LIO using overhead space above the line of sight of the doctor and patient. In one example, the compact housing has a volume below 2 dm³ (while the smallest system currently available is 29 dm³) and a total weight (including all enclosed components of the device 102) of less than 2 kg.

The control module 414 controls the treatment laser energy delivered by the laser module 112 based on parameter information received from the mobile computing device 104 and activation signals received from the activation device 106. In response to receiving the parameter information from the mobile computing device 104, the control module 414 sets the parameters for the treatment laser energy to be emitted by the laser module 112 and for the patterns to be produced by the scanning module 122. In response to receiving activation signals from the activation device 106, the control module 414 sends control signals reflecting the user-provided parameters to the laser module 112 activating the laser module and causing it to produce and/or emit the treatment laser energy and sends control signals to the scanning module 122 causing the scanning module 122 to reflect the laser energy 52 at different angles based on the selected pattern. In the illustrated example, the control module 414 is enclosed within the compact housing 400 of the pattern-scanning laser device 102. Prior to receiving the activation signal from the activation device 106, the control module 414 sends control signals to the laser module 112 activating the laser module and causing it to produce and/or emit the aiming laser beam and sends control signals to the scanning module 122 causing the scanning module 122 to reflect the laser energy 52 of the aiming laser beam at different angles so as to deliver the visible outline pattern indicating the treatment target area currently targeted by the apparatus, so as to allow the physician to adjust the aiming and direct the treatment to a desired treatment target area.

In the illustrated example, the control module 414 is enclosed within the compact housing 400 of the pattern-scanning laser device 102. In other embodiments, the control module 414 is external to the housing 400 and sends control signals to the laser module 112 and scanning module 122 via a data port.

The cooling mechanism 402 is attached to the laser module 112 and includes a baffle to provide passive cooling of the laser module 112 and/or a cooling fan.

The jumper 404 and emergency off switch 406 are safety mechanisms that shut down the laser module 112 under certain conditions. The jumper 404 includes an interlock circuit that must be closed (e.g. with a key switched on and/or a connector plugged into a jack) for the laser module 112 to operate. The emergency off switch 406 shuts down the laser module 112 in response to activation of a switch mechanism (e.g. by a user).

The F-theta lens 408 converts laser beam reflected from the scanning module 122 at different scan angles into patterns of spots focused at a single plane by redirecting the beams so that they are displaced from each other (based on the scan angle) but parallel with respect to each other. In general, F-theta lenses are designed with a barrel distortion that yields a displacement that is linear with the angle with respect to the lenses' optical axis.

The spot size selector module 410 is an optical system comprising multiple lenses, which move (e.g. via a motor) with respect to each other in order to adjust the spot size for each beam of the patterned laser energy 54. In one example, the spot size selector module 410 wirelessly receives control signals (e.g. from the control module 414 or other control mechanism) and adjusts the spot sizes based on the control signals.

The focusing lens directs the patterned laser energy 54 onto a focal plane.

It will be appreciated that other embodiments may include additional or alternative components.

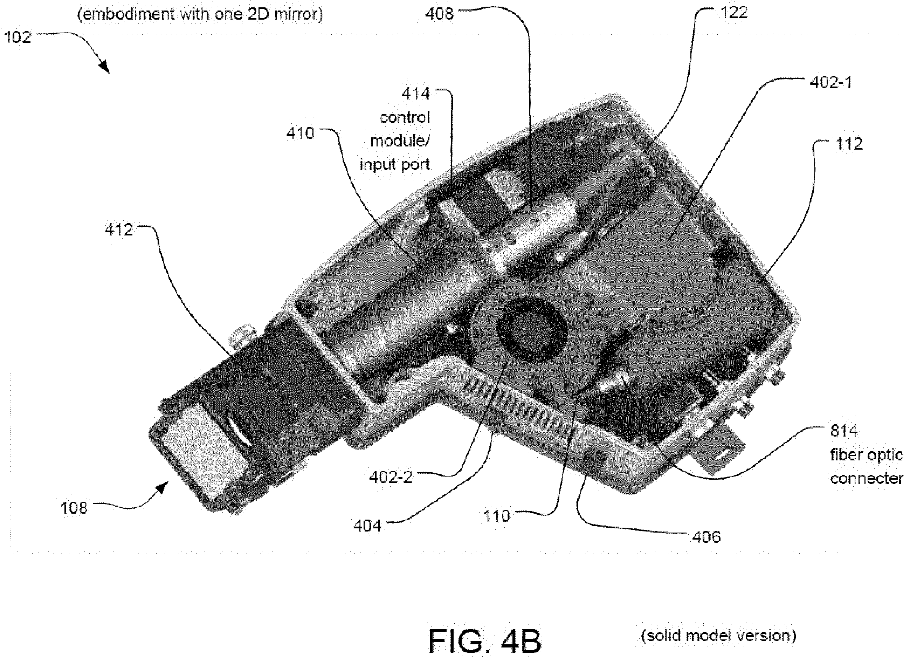

Fig. 4B is a perspective illustration of the pattern-scanning laser device 102 with a portion of the housing 400 cut away to reveal internal components.

The pattern-scanning laser device 102 includes the laser module 112, fiber optic cable 110 that ends with a collimation lens, scanning module 122, cooling mechanism 402, jumper 404, emergency off switch 406, control module 414, housing 400, and delivery optics 108, including the F-theta lens 408, spot size selector module 410, and focusing lens 412, as previously described.

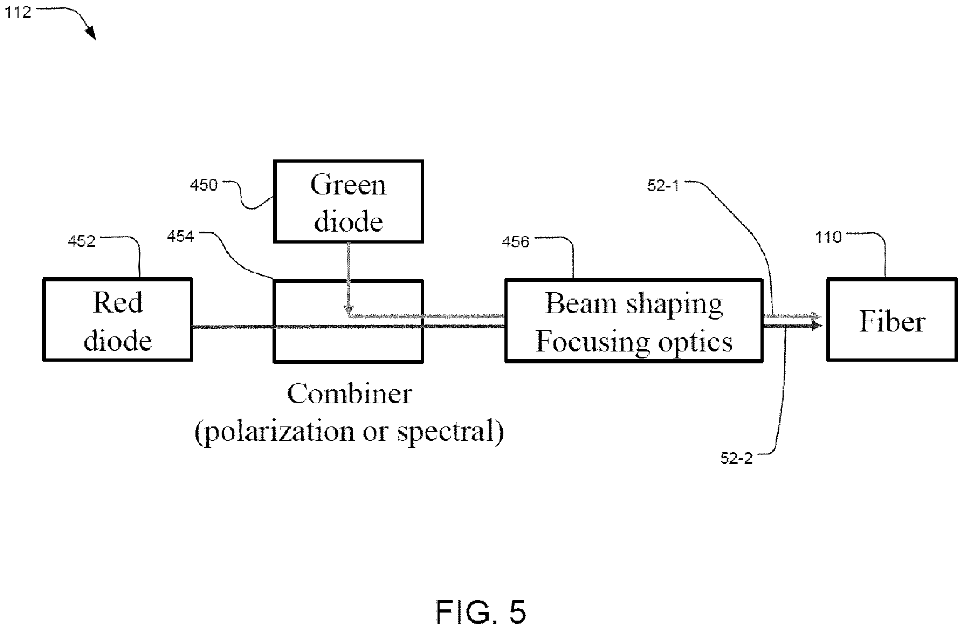

Fig. 5 is a schematic diagram of the laser module 112.

The laser module includes a treatment laser diode 450, e.g. a green laser diode, and an aiming laser diode 452, e.g. a red laser diode. It will be appreciated that other embodiments may include other types of laser sources. The laser module further comprises a combiner 454 and a focusing lens 456.

The laser diodes 450, 452 produce laser beams of particular wavelengths. For example, the treatment laser diode 450 may produce a laser beam in the wavelength range of 495-580 nm associated with visible green and yellow light. The aiming laser diode 452 may produce a laser beam in the wavelength range associated with visible red light for producing an aiming beam. The treatment laser beam 52-1 produced by the treatment laser diode 450 and the aiming laser beam 52-2 produced by the aiming laser diode 452 are combined by the polarization or spectral combiner (dichroic mirror) 454, which reflects one of the beams while transmitting the other, resulting in both propagating in the same direction. The combiner 454 directs the combined beam through the focusing lens 456 to the fiber optic cable 110 via a fiber optic connecter 814. It will be appreciated that other embodiments may comprise other means for combining and/or directing and delivering the respective laser beams.

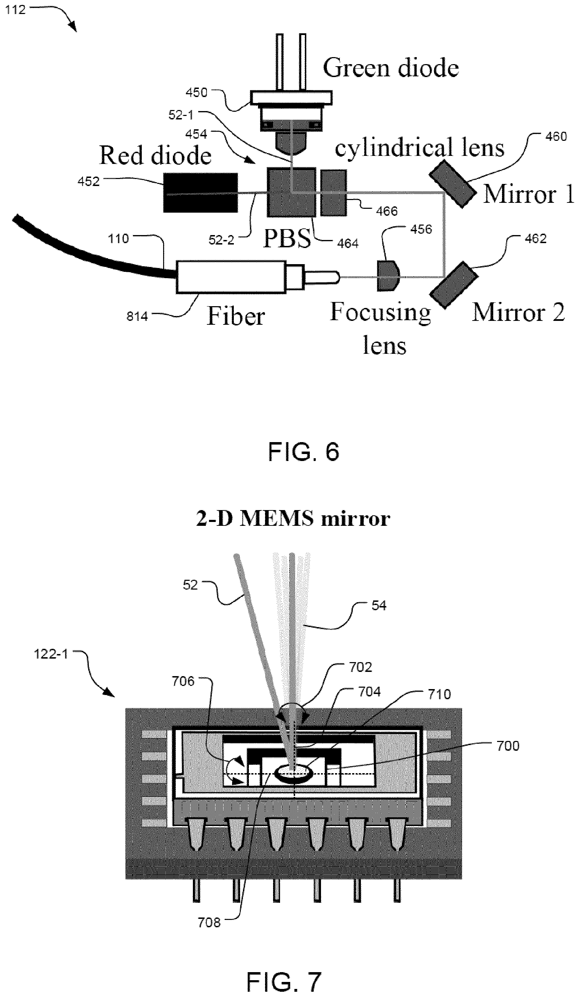

Fig. 6 is a schematic diagram of the laser module 112 showing the components in more detail.

The laser module 112 includes the target laser diode 450, the aiming laser diode 452, combiner 454, and focusing lens 456, as previously described.

Now, however, the components are shown in more detail, including a polarizing beam splitter 464 and a cylindrical lens 466 of the combiner 454, and a first mirror 460 and a second mirror 462 for compactness.

The treatment laser beam 52-1 produced by the treatment laser diode 450 and the aiming laser beam 52-2 produced by the aiming laser diode 452 are combined by the polarizing beam splitter 464, which reflects one of the beams while transmitting the other, resulting in both propagating in the same direction. The polarizing beam splitter 464 directs the combined beam through the cylindrical lens 466 (e.g. cylindrical telescope) to shape the beam. The first mirror 460 reflects the beam (e.g. at an angle of 90 degrees) to the second mirror 462, which reflects the beam (e.g. at an angle of 90 degrees) through the focusing lens 456 to the fiber optic cable 110 via the fiber optic connector 814.

This arrangement of the components of the laser module 112, including the two mirrors 460, 462 for redirecting the beam allows for a compact design of the laser module 112.

Fig. 7 is a perspective view of an exemplary scanning module 122-1 comprising a single two-dimensional scanning mirror 700.

In general, in some embodiments, the scanning module 122 is a microelectromechanical system (MEMS) microscanner containing a small mirror that has controllable tilt in one or two dimensions. The scanning module 122 comprises position sensors (e.g. piezo resistive position sensors), actuators for moving the scanning mirror 700 (e.g. piezoelectric, electrostatic or magnetostrictive actuators) and a controller (e.g. microprocessor) for controlling the actuators based on data from the position sensors.

In one embodiment, the scanning module 122 is a quasi-static or resonant two-dimensional MEMS scanner with vertical electrostatic comb drives and a MEMS fabrication process that is CMOS compatible.

In the illustrated example, the scanning mirror 700 rotates in a first direction 702 on a first axis 704 and also rotates in a second direction 706 on a second axis 708.

The scanning mirror comprises a reflective surface 710, which reflects incoming laser energy at different angles, for example, based on tilt of the mirror on each axis. The reflective surface 710 includes a reflective coating (e.g. HR Bragg coatings for 520 nm wavelengths or external optical HR coatings).

In the preferred embodiment, the scanning mirror 700 is a dielectric Bragg mirror with a reflective surface 710 comprising multiple thin layers of dielectric material with specified reflectivity at different wavelengths such that the scanning mirror 700 reflects the laser energy 52 with an absorption rate that is low enough to preserve the integrity of the mirror and prevent overheating.

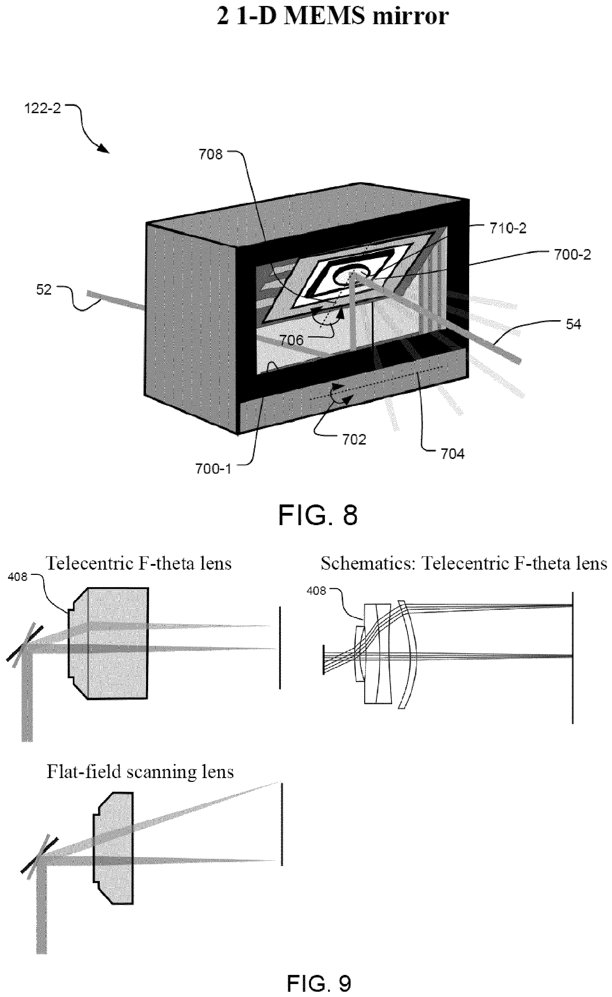

Fig. 8 is a perspective view of an exemplary scanning module 122-2 comprising two one-dimensional scanning mirrors 700.

The first scanning mirror 700-1 rotates in the first direction 702 around the first axis 704, reflecting light to the second scanning mirror 700-2, which rotates in the second direction 706 around the second axis 708.

Fig. 9 is a side view showing an exemplary F-theta lens 408. The F-theta lens 408 converts incoming deflected laser beams such that the focal plane is a flat surface. In the illustrated example, the F-theta lens 408 is a telecentric lens, which is designed such that the incoming laser beams strike normal to the work surface (e.g. the surface of the eye 50 of the patient) over the entire scanning field.

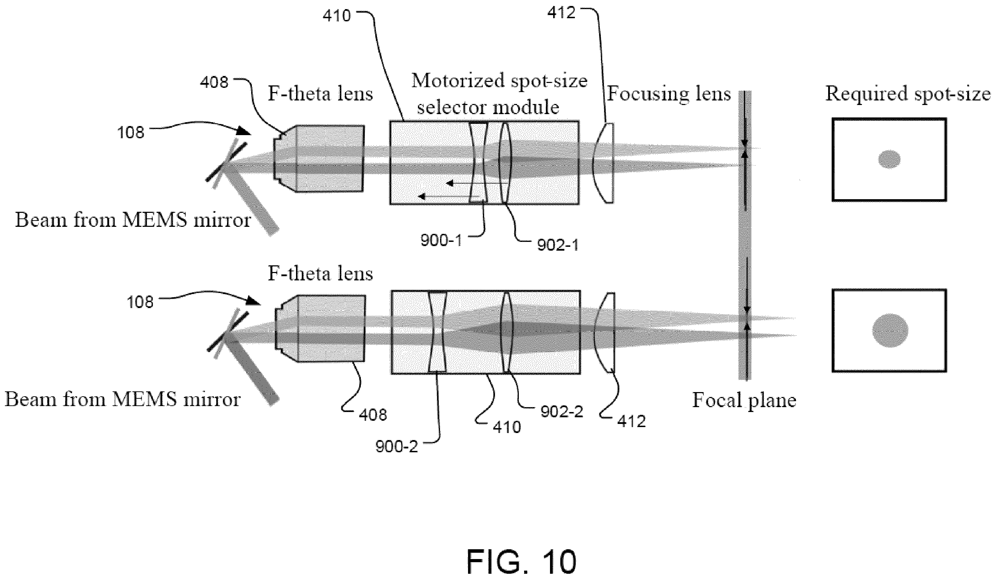

Fig. 10 is a side view of the delivery optics 108, including the F-theta lens 408, the spot-size selector module 410 and the focusing lens 412.

The spot-size selector module 410 includes a first selector biconcave lens 900-1 and a second selector biconvex lens 900-2. The F-theta lens 408 directs the patterned laser energy 54 first through the first selector lens 900-1 and then through the second selector lens 900-2 into the focusing planoconvex lens 412. The spot size is based on the distance between the selector lenses 900, 902.

In the top example, the first selector lens 900-1 is relatively close to the second selector lens 902-1, resulting in a relatively small spot size.

In the bottom example, the first selector lens 900-2 is relatively far from the second selector lens 902-2, resulting in a relatively larger spot size.

The spot-size selector module 410 operates by moving the first selector lens 900-1 along the lens system axis at varying distances from the second selector lens 900-2 based on the desired spot size. This movement is provided, for example, via a motorized movement mechanism which is wirelessly controlled.

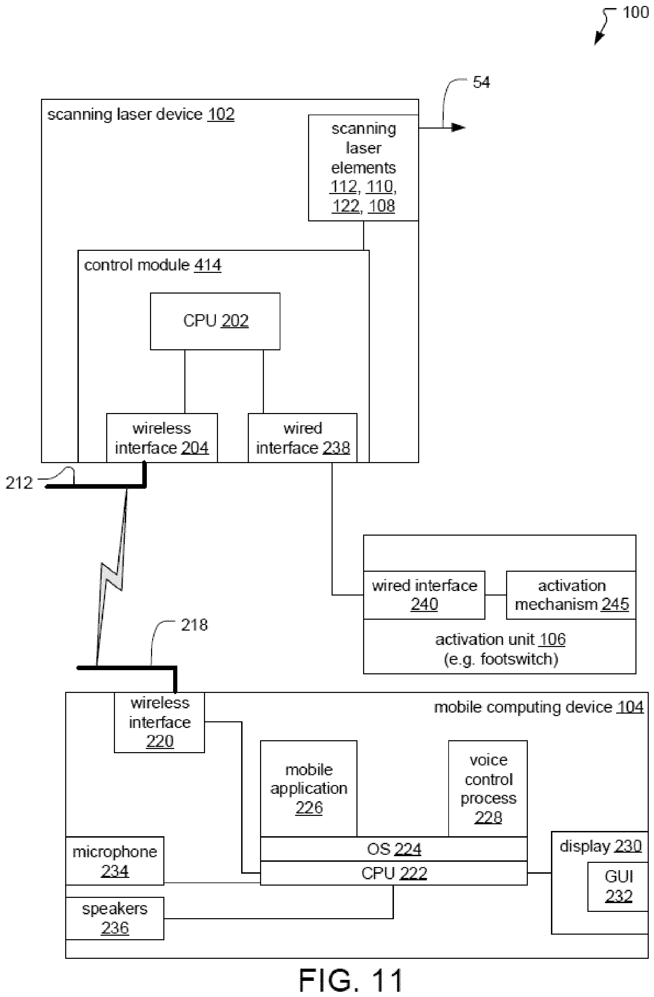

Fig. 11 is a schematic diagram of the laser treatment system 100 showing components of the scanning laser device 102, activation unit 106 and mobile computing device 104 in more detail.

The scanning laser device 102 includes the scanning laser elements 112, 110, 122, 108 and control module 414 as previously described.

Now, however, internal components of the control module 414 are shown.

The control module 414 includes a CPU 202, a wireless interface 204 and a wired interface 238. The CPU 202 directs the functionality of the control module 414 such as receiving parameter information from the mobile computing device 104 and activation signals from the activation device 106 via the wireless interface 204 and antenna 212 and the wired interface 238, as well as sending control signals to the laser module 112, the scanning module 122 and/or the spot-size selector module 410.

The mobile computing device 104 includes a central processing unit (CPU) 222, a touchscreen display 230, a wireless interface 220 and antenna 218, a microphone 234 and speakers 236.

The CPU 222 executes firmware/operating system instructions and sends instructions and data to and receives data from the wireless interface 220, the microphone 234, the speakers 236, and the display 230. Executing on typically an operating system (OS) 224 of the CPU 222 are a mobile application 226 and a voice control module 228. The mobile application 226 renders a graphical user interface (GUI) 232 on the touchscreen display 230.

The GUI 232, which is part of the user interface of the laser treatment system 100, is optimized to each treatment protocol and presents parameter information including stored pre-set treatment parameters for most used treatments, data from recent treatments, and/or a user manual, among other examples. The GUI 232 also receives parameter information, for example, by detecting contact between the user and the touchscreen display 230 in certain regions of the touchscreen display 230. The mobile application 226 also performs functions related to configuring the laser treatment system 100 such as pairing the mobile computing device 104 with the control module 414 and/or setting a wake word, which is a selected phrase for indicating that verbal commands follow.

The microphone 234 captures sound including the wake word and voice commands indicating parameter information provided by the user, which the mobile computing device 104 converts to audio data.

The speakers 236 produce sound based on instructions from the parameter regulation module 229, for example, in order to provide audible feedback confirming parameter information and/or voice commands.

In general, the voice control module 228 provides voice control capability, including internet-free connectivity and an expandable, customized library of multilingual commands. The voice control module 228 generates voice command information based on the captured audio data. In one example, the voice control module 228 recognizes spoken language in the audio data and translates the spoken language to commands that can be interpreted and/or executed by the control module 414.

In the illustrated example, the voice control module 228, microphone 234, speakers 236, GUI 232 rendered on the touchscreen display 230, and the activation device 106 provide a general user interface (UI) for the laser treatment system 100. However, in other embodiments (not illustrated) the UI for the laser treatment system 100 can also include other user interface elements such as physical input mechanisms such as knobs or buttons, which can be part of the mobile computing device 104 itself or part of peripheral devices connected to the mobile computing device 104 via the wireless interface 220 and/or a physical interface (e.g. data port). In general, the parameter information can be generated by the mobile computing device 104 based on any user engagement with the mobile computing device 104 and/or peripheral devices.

The wireless network interface 220 facilitates sending the parameter information to the control module 414 via the antenna 218 through a wireless communication link with the control module 414 according to wireless personal area network (WPAN) or wireless local area network (WLAN) protocols such as Bluetooth Low Energy (BLE) or WiFi, among other examples.

Finally, the activation unit 106 receives user input via an activation mechanism 245 (e.g. a switch or button) and in response to the user input, the activation unit 106 generates and sends activation signals to the control module 414 and/or to the mobile computing device 104, based on the configuration of the laser treatment system 100, via a wired interface 240. In the preferred embodiment, the activation unit 106 is a footswitch, and engagement with the activation mechanism 245 includes compression of the footswitch by the user's foot, for example.

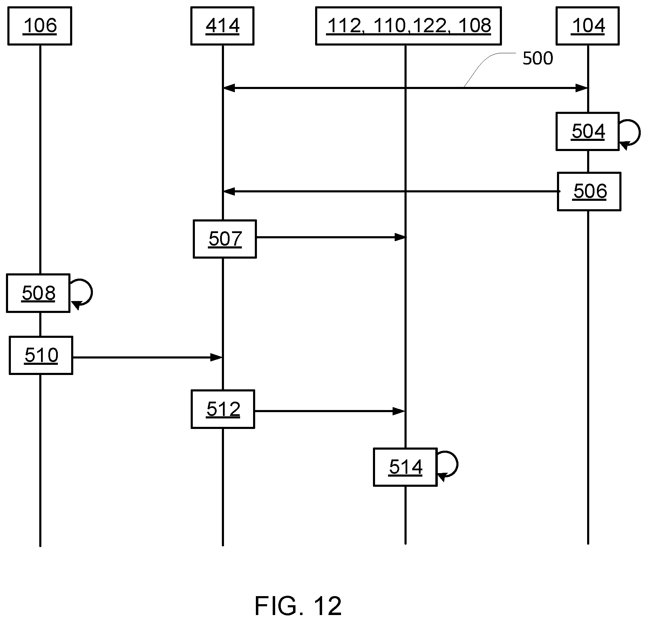

Fig. 12 is a sequence diagram illustrating the process by which the laser treatment system 100 delivers the patterned laser energy 54 to the eye 50 of the patient.

First, in step 500, the control module 414 pairs with the mobile computing device 104 by, for example, establishing a wireless communication link and/or exchanging identification information for the two devices, among other examples.

In step 504, the mobile computing device 104 detects input via the GUI 232 or the UI in general and generates parameter information based on the input. In one example, the doctor selects a virtual button indicating the power parameter or enters via a virtual keyboard a numerical value indicating the desire power setting (e.g. 200). In another example, the doctor adjusts a dial or increment button indicating the desired power setting (e.g. 200). In another example, the doctor speaks a desired power setting. In yet another example, the doctor indicates the desired pattern via the GUI 232 by selecting from a set of pre-determined patterns and/or by indicating where each spot in the pattern should be relative to the others, among other examples.

In step 506, the mobile computing device 104 sends the parameter information to the control module 414 along with instructions to set the required parameters based on the parameter information, and the parameters are updated.

In step 507, the control module 414 sends control signals to the pattern scanning laser elements, including the laser module 112, the scanning module 122, and/or the spot-size selector module 410. The control module 414 sends control signals to the laser module 112 indicating that the laser module should activate the aiming laser beam. The control module 414 also sends control signals to the scanning module 122 indicating that the scanning mirror(s) 900 should be tilted in a particular sequence (e.g. of tilt angles) so as to deliver a visible outline pattern indicating a periphery of the presently targeted treatment target area. The control module 414 further sends control signals to the laser module to selectively turn the aiming laser diode on and off in appropriate synchronism with the scanning of the scanning module so as to provide the visible outline pattern on the retina of the subject such that the visible outline pattern includes one or more pattern elements, each pattern element perceivable as moving along the periphery, as described herein.

In step 508, the activation unit 106 detects engagement of the activation mechanism 245. In one example, the user's foot compresses a footswitch. In response, in step 510, the activation unit 106 sends an activation signal to the control module 414.

In response to receiving the activation signal, the control module 414 in step 512 sends control signals to the pattern scanning laser elements, including the laser module 112, the scanning module 122, and/or the spot-size selector module 410. In one example, the control module 414 sends control signals to the laser module 112 indicating that the treatment laser diode should generate a treatment laser beam with a certain power level based on the parameter information. In another example, the control module 414 sends control signals to the scanning module 122 indicating that the scanning mirror(s) 900 should be tilted in a particular sequence (e.g. of tilt angles) based on a desired pattern indicated in the parameter information. In yet another example, the control module 414 sends control signals to the spot-size selector module 410 indicating adjustment of the spot size of the laser beams to a particular size based on the parameter information. In some embodiments, the control module further sends control signals to the laser module 112 indicating that the laser module should deactivate the aiming laser beam for the duration of the delivery sequence of the treatment laser beam and, optionally, to again activate the aiming laser beam upon completion of the treatment sequence.

In step 514, in response to receiving the control signals, the scanning laser elements generate and deliver the patterned treatment laser beam based on the control signals and the parameter information. For example, in response to receiving the control signals, the laser module 112 produces laser energy with a power and pulse sequence based on the control signals. In another example, in response to receiving the control signals, the scanning module 122 tilts the scanning mirror(s) 900 in order to reflect the laser energy 52 to the delivery optics 108 at different angles based on a predetermined pattern indicated by the control signals. In yet another example, the spot size selector module 410, in response to receiving the control signals, adjusts the spot size (e.g. by adjusting the positions of the first and second selector lenses 900, 902 relative to each other).

Subsequently, the control unit may return to step 507 and the activation unit may return to step 508, i.e. the process may again deliver the visible outline pattern so as to allow the physician to target another treatment target area, and the process may await a further activation of the activation mechanism.

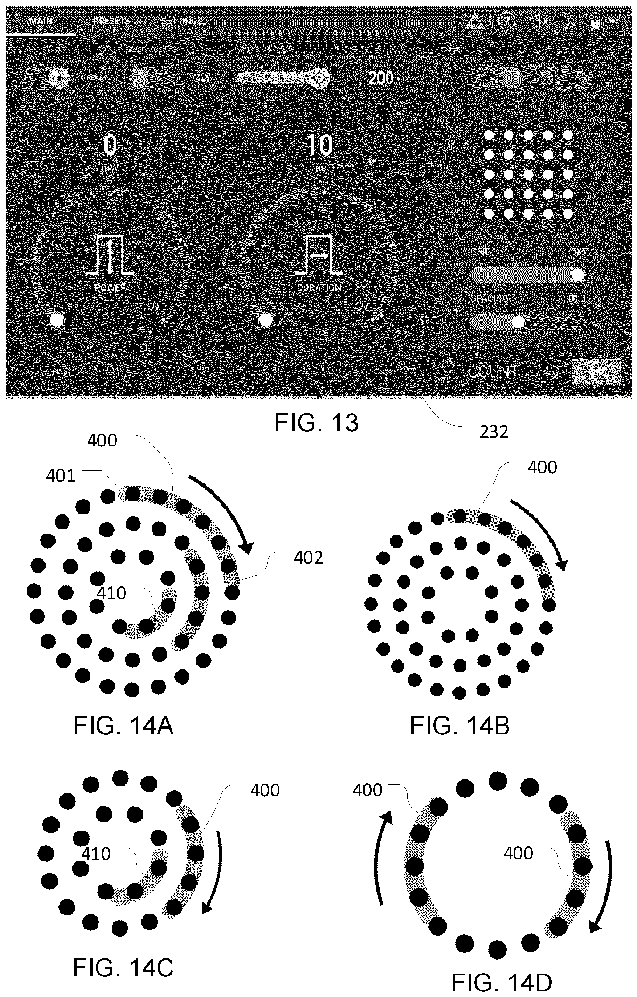

FIG. 13 shows an example of a graphical user interface 232 which allows a physician to select various parameters pertaining to the treatment, as described herein. In particular, the graphical user interface allows the user to select a treatment pattern of treatment locations from various pre-defined treatment patterns. Alternatively or additionally, the user interface may allow the physician to modify predefined treatment patterns and/or define new treatment patterns. For example, the user interface may provide functionality for allowing a user to select a type of pattern, e.g. square grid, treatment locations located on concentric circles, treatment locations located on segments of concentric circles, and/or the like. The user interface may further allow the physician to select one or more parameters defining attributes of the treatment pattern. Examples of such attributes include, a grid size, a spacing between treatment patterns. For example, the grid size may define whether a square grid is a 3x3 grid, a 4x4 grid, whether a pattern defined by one or more concentric circles includes one, two or more circles, etc.

The user interface may further provide functionality that allows a user to select other treatment parameters, e.g. a power level, a duration etc. of the individual doses of laser energy directed to the individual treatment locations, etc.

Further, the user interface may provide functionality that allows a user to adjust the parameters of the visible outline pattern, e.g. the number of lines or other pattern elements to be projected, the movement speed of the perceived movement of the pattern elements, the relative length of the lines (or size of other pattern elements) relative to the length of the periphery of the treatment area, and/or the like.

FIGs. 14A-D illustrate examples of treatment patterns and associated visible outline patterns. Generally, the treatment patterns include a plurality of treatment locations, in the form of laser spots, illustrated by black dots in the following drawings. The treatment locations are arranged in a user-selected pattern. In the examples of FIGs. 14A-D, the treatment locations are distrusted along one or more circles. The visible outline patterns are illustrated as grey lines. In particular, each visible outline pattern is formed by one or more separate and spaced apart pattern elements 400. In the present example, the pattern elements are lines. In the examples of FIGs. 14A-D, the lines are arc-shaped lines. In the example of FIG. 14A, the treatment locations are distributed on three concentric circles of respective radii. Visible outline pattern includes a single pattern element 400 in the form of a line extending along the outermost circle and intersecting a subset of the treatment locations on the outermost circle. The scanning module is controlled such that the aiming laser beam scans along a part of the outer circle while the aiming laser diode is turn on. This way, the human vision perceives an illuminated curved line coinciding with a portion of the outer circle. The scanning module is controlled such that it repeatedly scans along at least a part of the outer circle. During each scan, the aiming beam is controlled to be activated while the beam path scans along a different part of the outer circle such that the resulting visible line is perceived by the human vision of a physician, who is observing the retina, as moving along the outer circle in a direction from a trailing end 401 towards a leading end 402 of the line, as indicated by a curved arrow in FIG. 14A. The perceived movement is an apparent motion that is caused by the short projection of the line, followed by another, subsequent short projection of the same (or a similar) line at a different position. Such a sequence of short projections is experienced by human vision as one figure that moves. This phenomenon is sometimes also referred to as beta movement. The scanning module may be controlled such that the perceived movement of the line 400 extends all the way around the outer circle such that the moving line outlines the periphery of the treatment pattern.

In the example of FIG. 14A, the scanning module is further controlled to provide similar moving lines along the inner circles so as to outline the peripheries of each circular sub-pattern. However, in other embodiments, the scanning module is controlled so as to only outline one of the concentric circles, e.g. the central circle or the outer circle, e.g. as illustrated in FIG. 14B.

It will be appreciated that various modifications to the moving outline pattern may be made. For example, as illustrated in FIG. 14C, the treatment pattern may include a different number of circles. In particular, in the example of FIG. 14C, the treatment pattern includes only two concentric circles, each of which is outlined by a respective visible moving line 400 and 410, respectively.

In the example of FIG. 14D, the treatment pattern includes only a single circle. However, in this example, the visible outline pattern comprises two separate, spaced apart lines 400 that, at each point in time, are visible along respective portions of the circle. Both lines are generated such that they are perceived as moving along the circle in a common direction and at the same speed, as indicated by the curved arrows. Accordingly, they appear to follow or chase each other along the periphery of the treatment pattern. It will be appreciated that, in alternative embodiments, the pattern elements may be created such that they are perceived to move in opposite directions and/or at different speeds. It will further be appreciated that, in the embodiments of FIGs. 14A-C, the visible outline patterns of one or more of the circles may include more than one pattern element.

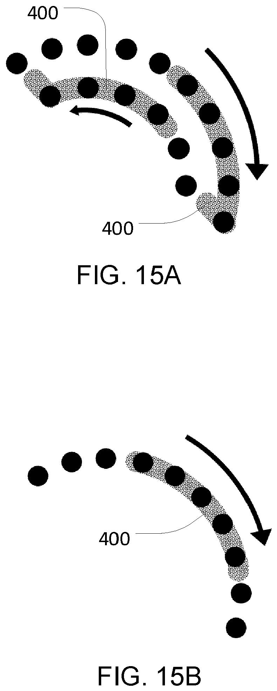

FIGs. 15A and 15B show examples of treatment patterns in the form of one or more circular arcs, and visible outline patterns indicating a periphery of the treatment target areas defined by the treatment patterns. In particular, in the example of FIG. 15A, the treatment locations form a treatment pattern including two circular arcs of respective concentric circles. The visible outline pattern has two lines 400 that are generated such that they are perceived to move along the periphery of the treatment area defined by the treatment pattern, as indicated by the curved arrows. In the example of FIG. 15B, the treatment pattern consists of a single circular arc on which the treatment locations are evenly distributed, i.e. the treatment area is narrow arcuate stripe. The visible outline pattern includes a single line 400 that is generated such that it is perceived to move along the arc in a direction indicated by the curved arrow. When the line reaches the end of the circular arc, it may reciprocate back along the arc in the opposite direction or it may disappear and reappear at the opposite end of the arc and again be perceived as moving along the arc in the same direction as before. This way, and since the line 400 has a certain width defined by the spot size of the aiming beam, the line 400 outlines the periphery of the arcuate treatment target area.

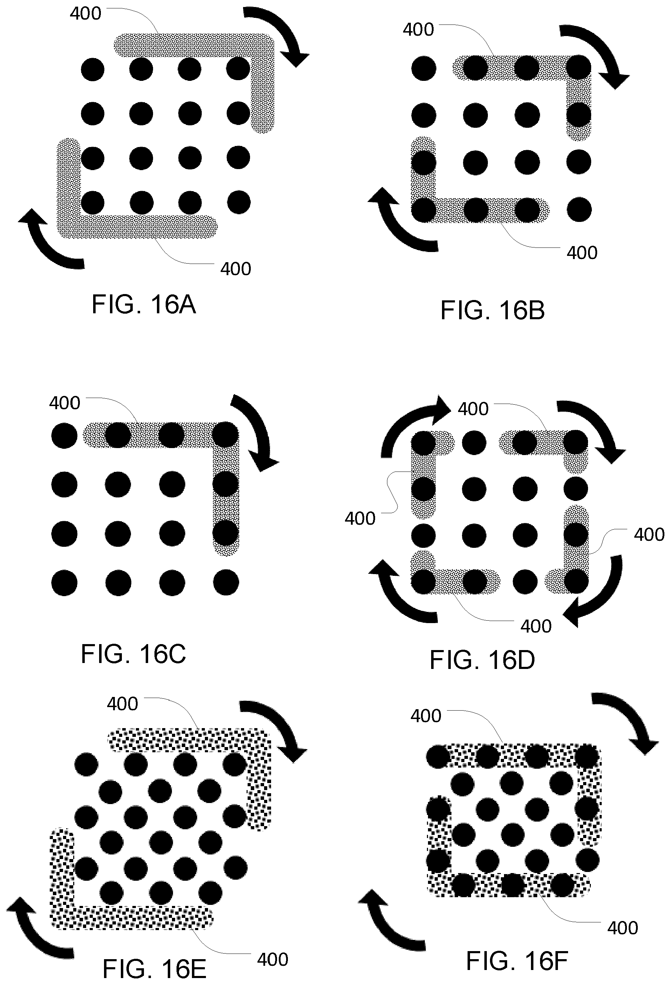

FIGs. 16A-F show examples of visible outline patterns that outline the periphery of a rectangular or square treatment target area, which includes a treatment pattern in the form of treatment locations arranged on a grid. In the example of FIGs. 16A-D, the grid is a 4x4 square grid. However, it will be appreciated that grids of other shapes and/or sizes are possible, including rectangular grids, or even grids having different unit cells, e.g. hexagonal grids, triangular grids, rhombic grids, etc., e.g. as illustrated in FIGs. 16E-F. In the examples of FIGs. 16A-B and 16E-F, the visible outline pattern has two pattern elements in the form of two lines 400 moving along the outer periphery of the treatment area defined by the grid. In the examples of FIGs. 16A and 16E, the lines 400 do not intersect any of the treatment locations, but they extend around, outside and adjacent to, the edges of the grid. In the examples of FIGs. 16B and 16F, the lines 400 intersect with the treatment locations on the outer edges of the grid. It will be appreciated that a visible outline pattern that does not intersect any of the treatment locations but extends outside of the treatment pattern may also be provide for the circular or arc-shaped treatment patterns of the previous examples.

In the example of FIG. 16C, the visible outline pattern includes only a single line 400 generated such that it is perceived as moving along the periphery of the square grid.

In the example of FIG. 16D, the visible outline pattern includes four separate and spaced apart lines 400 that are generated such that they appear to move along the periphery of the treatment area defined by the grid.

In the examples of FIG. 16C-D, the lines intersect the outer treatment locations, but it will be appreciated that the lines of FIG. 16C-D may also be provided such that they extend outside of the square patterns similar to the lines of FIG. 16A.

Generally, it will be appreciated that the lengths of the individual lines and the gaps between them may be varied. Larger gaps, e.g. as in FIG. 16C, allow an unobstructed view onto a larger portion of the retina. However, the gaps should be small enough so as to allow the physician an accurate assessment of the periphery. Similarly, the speed and/or direction of the perceived movement may be varied and/or other variations are possible, e.g. perceived movements that change direction e.g. so as to be perceived as reciprocating movement.

In this respect it should be borne in mind, that the treatment locations indicated by the black dots in the figures are in fact not visible to the physician during the process of positioning the visible outline pattern at a desired treatment target location, i.e. the treatment locations are not visible to the physician when the visible outline pattern is delivered. The treatment laser diode is normally switched off or deactivated while the physician views the visible outline pattern and positions the visible outline patterns at a position on the retina where the physician intends to perform the subsequent treatment.

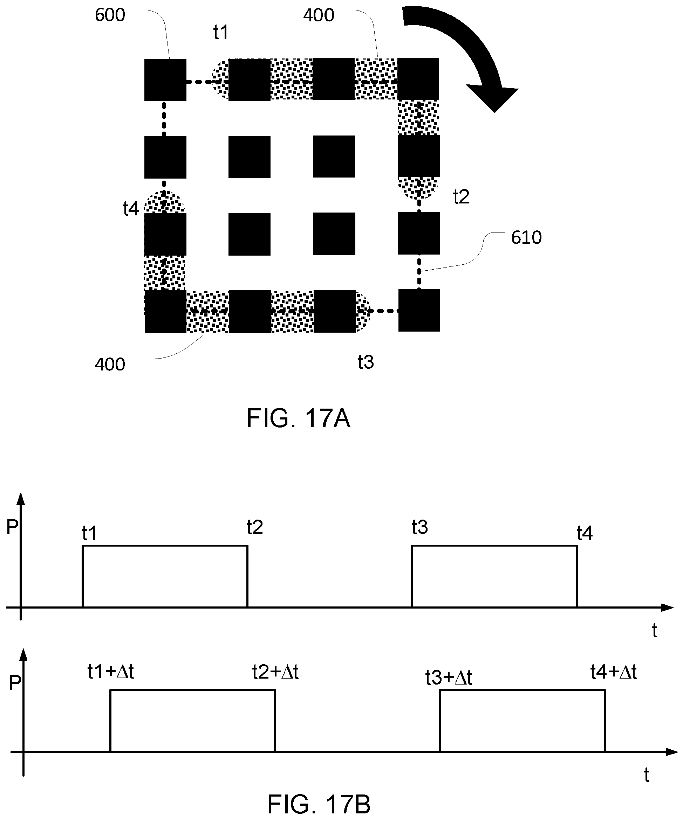

FIGs. 17A-B illustrate an example of how a visible outline pattern of pattern elements that are perceived as moving along the periphery of a treatment target area is generated by the scanning module.

In particular, FIGs. 17A-B illustrate the generation of the visible outline pattern of the example of FIG. 16C, which is reproduced in FIG. 17A. It will be appreciated that other visible outline patterns may be generated in an analogous manner. The visible outline pattern of FIG. 17A may be generated by controlling the scanning module to let the beam path of the aiming beam scan along the periphery of the treatment pattern. Each scan may start at position 600 and proceed along scan path 610 until the beam path is again directed toward position 600 and a subsequent scan along scan path 610 starts. Accordingly, when the start of each scan at point 600 marks a time t = 0, each position along the scan path 610 corresponds to a respective time, as illustrated by times t1,..., t4 in FIG. 17A, where 0 < t1 < t2 < t3 < t4. During each scan of the scanning module, the aiming laser diode is controlled to be turned on only during a part of each scan. It will be appreciated that, instead of turning the laser diode on and off during each scan, the aiming beam may be selectively activated and deactivated in a different manner. For example, a shutter may be employed to selectively block the aiming laser beam during a part of the scan.

FIG. 17B illustrates the power of the aiming beam during each scan. The upper curve illustrates the power of the aiming beam during a first scan along scan path 610, the scan starting and finishing at point 600, while the bottom curve illustrates the power during a subsequent scan along scan path 610, again starting and finishing at point 600. During the first scan, when the scan starts at time t = 0 and position 600, the aiming laser beam is initially off. It is turned on at time t1 and left on until time t2 where it is again turned off. At time t3, the aiming laser diode is again turned on und left on until time t4 where the aiming laser diode is turned off again. This repeated selective turning on and off of the aiming laser while the scanning module scans the beam path along the periphery of the treatment pattern causes the human vision of the physician, who is observing the retina during the scanning operation, to perceive two illuminated lines 400 that extend along respect portions of the periphery of the intended treatment pattern, as illustrated in FIG. 17A. In particular, the scanning speed at which the aiming laser beam scans along the beam path is selected sufficiently high, the lines 400 are perceived by human vision as substantially uniformly illuminated lines rather than as a moving spot.

During the subsequent scan of the beam path along the periphery 610, again starting from position 600, the aiming laser diode is again selectively turned on and off at respective points along the scan path. However, during the subsequent scan, the switching times are shifted by a predetermined delay Δt relative to the switching times of the previous scan. Accordingly, during the subsequent scan, the aiming laser is turned on at time t1 + Δt, turned off again at time t2 + Δt, turned on again at time t3 + Δt and turned off again at time t4 + Δt. Accordingly, the physician observing the retina during the scanning operation again perceives two illuminated lines 400 that extend along respect portions of the periphery of the intended treatment pattern. However, this time, the lines appear displaced relative to the previous scan. This process is repeated such that during the next scan, the switching times are again delayed by the delay Δt, i.e. the aiming laser diode is turned on at t1 + 2Δt and so on.

The scanning speed and the delay Δt are chosen such that the illuminated lines appearing at continuously changing positions are perceived by human vision of a human observer as lines moving along the periphery of the treatment pattern.

In some embodiments, the apparatus is configured to deliver the treatment beam to treatment locations defining a user-selected treatment pattern, wherein the visible outline pattern includes a number of pattern elements, wherein the number of pattern elements is indicative of the user-selected treatment pattern. For example, the number of pattern elements of the visible outline pattern may be chosen to be indicative of the size or shape of the treatment pattern and/or indicative of the number of treatment locations in the treatment pattern.

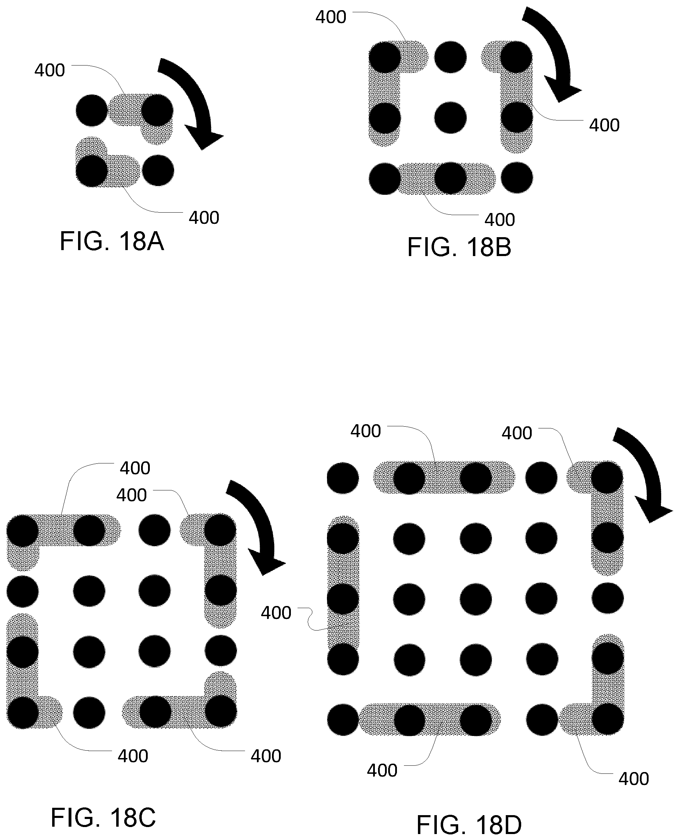

FIGs. 18A-D show examples of visible outline patterns that outline the periphery of a square treatment target area, which includes a treatment pattern in the form of treatment locations arranged on a grid. In the example of FIGs. 18A-D, the grid is a NxN square grid. However, it will be appreciated that grids of other shapes are possible, including rectangular grids, or even grids having different unit cells, e.g. hexagonal grids, triangular grids, rhombic grids, etc. In the examples of FIGs. 18A-D, the visible outline pattern has a number of pattern elements that is equal to the size of the grid, i.e. N pattern elements indicative of an NxN grid. The pattern elements are in the form of lines 400 moving along the outer periphery of the treatment area defined by the grid. FIG. 18A shows a treatment pattern in the form of a 2x2 grid of treatment locations and a visible outline pattern having 2 lines 400. FIG. 18B shows a treatment pattern in the form of a 3x3 grid of treatment locations and a visible outline pattern having 3 lines 400. FIG. 18C shows a treatment pattern in the form of a 4x4 grid of treatment locations and a visible outline pattern having 4 lines 400, and FIG. 18D shows a treatment pattern in the form of a 5x5 grid of treatment locations and a visible outline pattern having 5 lines 400. However, it will be appreciated that other types of pattern elements may be used. Similarly, the number of pattern elements may indicate another parameter of the treatment pattern, other than the grid size or number of treatment locations. For example, the number of pattern elements may indicate the shape of the treatment pattern, e.g. three pattern elements may indicate a triangular treatment pattern while four pattern elements may indicate a square or rectangular treatment pattern and/or the like. Accordingly, the operator is provided with a convenient indication/confirmation of the type of selected treatment pattern during the aiming stage, i.e. prior to delivering the treatment beam. Moreover, the operator does not need to divert attention away from the treatment area to be provided with the information. It will be appreciated that the number of displayed pattern elements may also be chosen to be indicative of another parameter of the treatment beam, e.g. a selected pulse duration, beam intensity, and/or the like. The type of information indicated by the number of pattern elements may be user-configurable or predetermined.

While this invention has been particularly shown and described with references to preferred embodiments thereof, it will be understood by those skilled in the art that various changes in form and details may be made therein without departing from the scope of the invention encompassed by the appended claims. It will be appreciated that other embodiments may include alternative or additional elements or features.

For example, it will be appreciated that treatment patterns and treatment target areas of various shapes and sizes may be used. Similarly, visible outline patterns of different shapes, e.g. using different types of pattern elements, may be used. For example, some visible outline patterns may include additional pattern elements in addition to the pattern elements that are perceived as moving along the periphery. For example, some visible outline patterns may include additional pattern elements that appear substantially stationary.

In the claims enumerating several means, several of these means can be embodied by one and the same element, component or item of hardware. The mere fact that certain measures are recited in mutually different dependent claims or described in different embodiments does not indicate that a combination of these measures cannot be used to advantage.

## Claims

1. An apparatus (100) for photothermal ophthalmic treatment comprising:
a) a treatment light source (450) for producing a treatment beam (54),
b) a scanning module (122) configured for delivering the treatment beam onto separate treatment locations within a treatment target area on a structure of a subject's eye (50),
c) an aiming light source (452) for producing an aiming beam,
wherein the scanning module is configured for delivering the aiming beam to create a visible outline pattern on the structure of the subject's eye, the visible outline pattern being indicative of a periphery of said treatment target area,
wherein the visible outline pattern includes one or more pattern elements (400), each pattern element perceivable as moving along the periphery, and
the scanning module is configured to cause an aiming beam path of the aiming beam to repeatedly scan along a scan path extending along the periphery, and
the apparatus is configured to selectively activate and deactivate the aiming beam so as to only illuminate respective portions of the scan path during each scan along the scan path, to create a perceivable beta movement of the visible outline pattern along the periphery.

2. An apparatus according to claim 1, wherein each pattern element is a line, shorter than the periphery, and extending along a portion of the periphery, the line having a leading end and a trailing end, displaced from the leading end.

3. An apparatus according to claim 2, wherein the line is perceivable as moving along the periphery in a direction between the trailing end and the leading end.

4. An apparatus according to claim 3, wherein the visible outline pattern includes two or more separate pattern elements which are spaced apart from each other.

5. An apparatus according to claim 3 or 4, wherein the separate pattern elements comprised in the visual outline pattern are perceivable as moving along the periphery in a common direction at a common speed.

6. An apparatus according to any one of the preceding claims, wherein the apparatus is configured:
- during a first scan of the aiming beam path along a scan path extending along the periphery, to selectively activate and deactivate the aiming beam so as to only illuminate a first portion of scan path,
- during a second scan of the aiming beam path along said scan path, subsequent to the first scan, to selectively activate and deactivate the aiming beam so as to only illuminate a second portion of the scan path, the second portion being displaced relative to the first portion along the direction of the scan path.

7. An apparatus according to any one of the preceding claims, comprising a user-interface (232) configured to provide functionality for user-selection of a treatment pattern, the treatment pattern defining relative locations of the plurality of separate treatment locations within a treatment target area and relative to each other.

8. An apparatus according to any one of the preceding claims, comprising a user-interface (232) configured to provide functionality for user-selection of one or more attributes of the visible outline pattern, the one or more attributes including one or more of a speed of the perceived movement of the one or more pattern elements, a number of pattern elements, a size of the one or more pattern elements, a shape of the one or more pattern elements.

9. An apparatus according to any one of the preceding claims, wherein the visual outline pattern covers between 20% and 90%, such as between 30% and 80% of the periphery.

10. An apparatus according to any one of the preceding claims, configured to select the number of pattern elements of the visible outline pattern responsive to a property of the delivery of the treatment beam.

11. An apparatus according to claim 10, wherein the property of the delivery of the treatment beam is a user-selected property.

12. An apparatus according to claim 10, wherein the apparatus is configured to deliver the treatment beam to treatment locations defining a user-selected treatment pattern, and wherein the apparatus is configured to select the number of pattern elements of the visible outline pattern responsive to the user-selected treatment pattern.

13. An apparatus according to any one of the preceding claims, wherein the apparatus comprises one or more of the following:
- a slit-lamp (101) and/or a slit-lamp adapter (102),
- a head-mounted device (103),
- a surgical microscope configured for use in an operating room or an adapter for a surgical microscope configured for use in an operating room.

14. A non-therapeutic method for providing a visible aiming pattern on a structure of a subject's eye (50), the method comprising delivering an aiming beam to create a visible aiming pattern on the structure of the subject's eye, the aiming pattern being a visible outline pattern indicative of a periphery of a treatment target area,
wherein the visible outline pattern includes one or more pattern elements (400), each pattern element perceivable as moving along the periphery,
and that the method comprises causing an aiming beam path of the aiming beam to repeatedly scan along a scan path extending along the periphery, and selectively activating and deactivating the aiming beam so as to only illuminate respective portions of the scan path during each scan along the scan path, to create a perceivable beta movement of the visible outline pattern along the periphery.

## Patentansprüche

1. Einrichtung (100) zur photothermischen Augenbehandlung, umfassend:
a) eine Behandlungslichtquelle (450) zum Erzeugen eines Behandlungsstrahls (54),
b) ein Abtastungsmodul (122), das zum Abgeben des Behandlungsstrahls auf separate Behandlungsstellen innerhalb eines Behandlungszielbereichs auf einer Struktur des Auges eines Probanden (50) konfiguriert ist,
c) eine Ziellichtquelle (452) zum Erzeugen eines Zielstrahls,
wobei das Abtastungsmodul zum Abgeben des Zielstrahls konfiguriert ist, um ein sichtbares Umrissmuster auf der Struktur des Auges des Probanden zu erstellen, wobei das sichtbare Umrissmuster einen Umfang des genannten Behandlungszielbereichs angibt,
wobei das sichtbare Umrissmuster ein oder mehrere Musterelemente (400) einschließt, wobei jedes Musterelement als sich entlang des Umfangs bewegend wahrnehmbar ist, und
das Abtastungsmodul dafür konfiguriert ist, zu bewirken, dass ein Zielstrahlpfad des Zielstrahls wiederholt entlang eines Abtastungspfades abtastet, der sich entlang des Umfangs erstreckt, und
die Einrichtung dafür konfiguriert ist, den Zielstrahl selektiv zu aktivieren und zu deaktivieren, um dadurch während jeder Abtastung entlang des Abtastungspfades nur jeweilige Abschnitte des Abtastungspfades zu beleuchten, um eine wahrnehmbare Beta-Bewegung des sichtbaren Umrissmusters entlang des Umfangs zu erstellen.

2. Einrichtung nach Anspruch 1, wobei jedes Musterelement eine Linie ist, die kürzer als der Umfang ist und sich entlang eines Abschnitts des Umfangs erstreckt, wobei die Linie ein vorderes Ende und ein hinteres Ende, das gegenüber dem vorderen Ende versetzt ist, aufweist.

3. Einrichtung nach Anspruch 2, wobei die Linie so wahrnehmbar ist, als bewegte sie sich entlang des Umfangs in einer Richtung zwischen dem hinteren Ende und dem vorderen Ende.

4. Einrichtung nach Anspruch 3, wobei das sichtbare Umrissmuster zwei oder mehr separate Musterelemente einschließt, die voneinander beabstandet sind.

5. Einrichtung nach Anspruch 3 oder 4, wobei die im sichtbaren Umrissmuster umfassten separaten Musterelemente so wahrnehmbar sind, als bewegten sie sich entlang des Umfangs in einer gemeinsamen Richtung mit einer gemeinsamen Geschwindigkeit.

6. Einrichtung nach einem der vorstehenden Ansprüche, wobei die Einrichtung dafür konfiguriert ist:
- während einer ersten Abtastung des Zielstrahlpfades entlang eines Abtastungspfades, der sich entlang des Umfangs erstreckt, den Zielstrahl selektiv zu aktivieren und zu deaktivieren, um nur einen ersten Abschnitt des Abtastungspfades zu beleuchten,
- während einer zweiten Abtastung des Zielstrahlpfades entlang des genannten Abtastungspfades, die auf die erste Abtastung folgt, den Zielstrahl selektiv zu aktivieren und zu deaktivieren, um nur einen zweiten Abschnitt des Abtastungspfades zu beleuchten, wobei der zweite Abschnitt relativ zum ersten Abschnitt entlang der Richtung des Abtastungspfades versetzt ist.

7. Einrichtung nach einem der vorstehenden Ansprüche, die eine Benutzerschnittstelle (232) umfasst, die dafür konfiguriert ist, Funktionalität zur Auswahl eines Behandlungsmusters durch Benutzer bereitzustellen, wobei das Behandlungsmuster relative Stellen der Vielzahl von separaten Behandlungsstellen innerhalb eines Behandlungszielbereichs und relativ zueinander definiert.

8. Einrichtung nach einem der vorstehenden Ansprüche, die eine Benutzerschnittstelle (232) umfasst, die dafür konfiguriert ist, Funktionalität zur Auswahl eines oder mehrerer Attribute des sichtbaren Umrissmusters durch Benutzer bereitzustellen, wobei das eine oder die mehreren Attribute eines oder mehrere von einer Geschwindigkeit der wahrgenommenen Bewegung des einen oder der mehreren Musterelemente, einer Anzahl von Musterelementen, einer Größe des einen oder der mehreren Musterelemente, einer Form des einen oder der mehreren Musterelemente einschließt.

9. Einrichtung nach einem der vorstehenden Ansprüche, wobei das sichtbare Umrissmuster zwischen 20 % und 90 %, wie etwa zwischen 30 % und 80 %, des Umfangs abdeckt.

10. Einrichtung nach einem der vorstehenden Ansprüche, die dafür konfiguriert ist, die Anzahl der Musterelemente des sichtbaren Umrissmusters als Antwort auf eine Eigenschaft der Abgabe des Behandlungsstrahls auszuwählen.

11. Einrichtung nach Anspruch 10, wobei die Eigenschaft der Abgabe des Behandlungsstrahls eine vom Benutzer ausgewählte Eigenschaft ist.

12. Einrichtung nach Anspruch 10, wobei die Einrichtung dafür konfiguriert ist, den Behandlungsstrahl an Behandlungsstellen abzugeben, die ein vom Benutzer ausgewähltes Behandlungsmuster definieren, und wobei die Einrichtung dafür konfiguriert ist, die Anzahl der Musterelemente des sichtbaren Umrissmusters als Antwort auf das vom Benutzer ausgewählte Behandlungsmuster auszuwählen.

13. Einrichtung nach einem der vorstehenden Ansprüche, wobei die Einrichtung eines oder mehrere von Folgendem umfasst:
- eine Spaltlampe (101) und/oder einen Spaltlampenadapter (102),
- eine am Kopf befestigte Vorrichtung (103),
- ein Operationsmikroskop, das für die Verwendung in einem Operationssaal konfiguriert ist, oder einen Adapter für ein Operationsmikroskop, das für die Verwendung in einem Operationssaal konfiguriert ist.

14. Nicht-therapeutisches Verfahren zum Bereitstellen eines sichtbaren Zielmusters auf einer Struktur des Auges eines Probanden (50), wobei das Verfahren Abgeben eines Zielstrahls umfasst, um ein sichtbares Zielmuster auf der Struktur des Auges des Probanden zu erstellen,
das Zielmuster ein sichtbares Umrissmuster ist, das einen Umfang eines Behandlungszielbereichs angibt,
wobei das sichtbare Umrissmuster ein oder mehrere Musterelemente (400) einschließt, wobei jedes Musterelement als sich entlang des Umfangs bewegend wahrnehmbar ist,
und wobei das Verfahren Bewirken umfasst, dass ein Zielstrahlpfad des Zielstrahls wiederholt entlang eines Abtastungspfades abtastet, der sich entlang des Umfangs erstreckt, und selektives Aktivieren und Deaktivieren des Zielstrahls, um dadurch während jeder Abtastung entlang des Abtastungspfades nur jeweilige Abschnitte des Abtastungspfades zu beleuchten, um eine wahrnehmbare Beta-Bewegung des sichtbaren Umrissmusters entlang des Umfangs zu erstellen.

## Revendications

1. Appareil (100) pour traitement ophtalmique photothermique comprenant :
a) une source de lumière de traitement (450) pour produire un faisceau de traitement (54),
b) un module de balayage (122) configuré pour délivrer le faisceau de traitement sur des emplacements de traitement distincts au sein d'une zone cible de traitement sur une structure de l'œil (50) d'un sujet,
c) une source de lumière de visée (452) pour produire un faisceau de visée,
dans lequel le module de balayage est configuré pour délivrer le faisceau de visée afin de créer un motif de contour visible sur la structure de l'œil du sujet, le motif de contour visible indiquant une périphérie de ladite zone cible de traitement,
dans lequel le motif de contour visible inclut un ou plusieurs éléments de motif (400), chaque élément de motif étant perceptible comme se déplaçant le long de la périphérie, et
le module de balayage est configuré pour amener une trajectoire de faisceau de visée du faisceau de visée à balayer de manière répétée le long d'une trajectoire de balayage s'étendant sur la périphérie, et
l'appareil est configuré pour activer et désactiver sélectivement le faisceau de visée afin d'éclairer uniquement des portions respectives de la trajectoire de balayage lors de chaque balayage le long de la trajectoire de balayage, afin de créer un mouvement bêta perceptible du motif de contour visible le long de la périphérie.

2. Appareil selon la revendication 1, dans lequel chaque élément de motif est une ligne, plus courte que la périphérie, et s'étendant le long d'une portion de la périphérie, la ligne présentant une extrémité avant et une extrémité arrière décalée par rapport à l'extrémité avant.

3. Appareil selon la revendication 2, dans lequel la ligne est perceptible comme se déplaçant le long de la périphérie dans une direction entre l'extrémité arrière et l'extrémité avant.

4. Appareil selon la revendication 3, dans lequel le motif de contour visible inclut deux ou plusieurs éléments de motif distincts qui sont espacés les uns des autres.

5. Appareil selon la revendication 3 ou 4, dans lequel les éléments de motif distincts compris dans le motif de contour visible sont perceptibles comme se déplaçant le long de la périphérie dans une direction commune à une vitesse commune.

6. Appareil selon l'une quelconque des revendications précédentes, dans lequel l'appareil est configuré :
- lors d'un premier balayage de la trajectoire de faisceau de visée le long d'une trajectoire de balayage s'étendant le long de la périphérie, pour activer et désactiver sélectivement le faisceau de visée afin d'éclairer uniquement une première portion de la trajectoire de balayage,
- lors d'un second balayage de la trajectoire de faisceau de visée le long de ladite trajectoire de balayage, après le premier balayage, pour activer et désactiver sélectivement le faisceau de visée afin d'éclairer uniquement une seconde portion de la trajectoire de balayage, la seconde portion étant déplacée par rapport à la première portion dans la direction de la trajectoire de balayage.

7. Appareil selon l'une quelconque des revendications précédentes, comprenant une interface utilisateur (232) configurée pour fournir une fonctionnalité de sélection par l'utilisateur d'un motif de traitement, le motif de traitement définissant des emplacements relatifs de la pluralité d'emplacements de traitement distincts dans une zone cible de traitement et les uns par rapport aux autres.

8. Appareil selon l'une quelconque des revendications précédentes, comprenant une interface utilisateur (232) configurée pour fournir une fonctionnalité de sélection par l'utilisateur d'un ou de plusieurs attributs du motif de contour visible, les un ou plusieurs attributs incluant un ou plusieurs parmi une vitesse du mouvement perçu des un ou plusieurs éléments de motif, un nombre d'éléments de motif, une taille des un ou plusieurs éléments de motif, une forme des un ou plusieurs éléments de motif.

9. Appareil selon l'une quelconque des revendications précédentes, dans lequel le motif de contour visible couvre entre 20 % et 90 %, par exemple entre 30 % et 80% de la périphérie.

10. Appareil selon l'une quelconque des revendications précédentes, configuré pour sélectionner le nombre d'éléments de motif du motif de contour visible en réponse à une propriété de la délivrance du faisceau de traitement.

11. Appareil selon la revendication 10, dans lequel la propriété de la délivrance du faisceau de traitement est une propriété sélectionnée par l'utilisateur.

12. Appareil selon la revendication 10, dans lequel l'appareil est configuré pour délivrer le faisceau de traitement à des emplacements de traitement définissant un motif de traitement sélectionné par l'utilisateur, et dans lequel l'appareil est configuré pour sélectionner le nombre d'éléments de motif du motif de contour visible en réponse au motif de traitement sélectionné par l'utilisateur.

13. Appareil selon l'une quelconque des revendications précédentes, dans lequel l'appareil comprend un ou plusieurs des éléments suivants :
- une lampe à fente (101) et/ou un adaptateur pour lampe à fente (102),
- un dispositif monté sur la tête (103),
- un microscope chirurgical configuré pour être utilisé dans une salle d'opération ou un adaptateur pour un microscope chirurgical configuré pour être utilisé dans une salle d'opération.

14. Procédé non thérapeutique destiné à fournir un motif de visée visible sur une structure de l'œil (50) d'un sujet, le procédé comprenant la délivrance d'un faisceau de visée pour créer un motif de visée visible sur la structure de l'œil du sujet,
le motif de visée étant un motif de contour visible indiquant une périphérie d'une zone cible de traitement,
dans lequel le motif de contour visible inclut un ou plusieurs éléments de motif (400), chaque élément de motif étant perceptible comme se déplaçant le long de la périphérie,
et dans lequel le procédé comprend le fait d'amener une trajectoire de faisceau de visée du faisceau de visée à balayer de manière répétée le long d'une trajectoire de balayage s'étendant le long de la périphérie, et l'activation et la désactivation sélective du faisceau de visée afin d'éclairer uniquement des portions respectives de la trajectoire de balayage lors de chaque balayage le long de la trajectoire de balayage, afin de créer un mouvement bêta perceptible du motif de contour visible le long de la périphérie.
